(19)

**Europäisches Patentamt**
**European Patent Office**
**Office européen des brevets**

(11) **EP 4 629 887 B1**

(12) **EUROPEAN PATENT SPECIFICATION**

(45) Date of publication and mention
of the grant of the patent:
**25.03.2026  Bulletin 2026/13**

(21) Application number: **22854772.5**

(22) Date of filing: **09.12.2022**

(51) International Patent Classification (IPC):
*A61B 5/02* $^{(2006.01)}$       *A61B 5/021* $^{(2006.01)}$
*G16H 50/50* $^{(2018.01)}$

(52) Cooperative Patent Classification (CPC):
**A61B 5/02007; A61B 5/02108; G16H 50/20;
G16H 50/50;** A61B 5/02028

(86) International application number:
**PCT/EP2022/085248**

(87) International publication number:
**WO 2024/120649 (13.06.2024 Gazette 2024/24)**

(54) **A METHOD FOR CALCULATION OF A PRESSURE DROP BETWEEN CROSS-SECTIONS OF AN ARTERY**

VERFAHREN ZUR BERECHNUNG EINES DRUCKABFALLS ZWISCHEN QUERSCHNITTEN EINER ARTERIE

PROCÉDÉ DE CALCUL D'UNE CHUTE DE PRESSION ENTRE DES SECTIONS TRANSVERSALES D'UNE ARTÈRE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(43) Date of publication of application:
**15.10.2025  Bulletin 2025/42**

(73) Proprietor: **Hemolens Diagnostics spolka z
ograniczona odpowied
54-202 Wroclaw (PL)**

(72) Inventor: **MIROTA, Kryspin
52-200 Wroclaw (PL)**

(74) Representative: **JWP Patent & Trademark
Attorneys
ul. Minska 75
03-828 Warszawa (PL)**

(56) References cited:
WO-A1-2021/222530       WO-A2-2019/175612
US-A1- 2013 246 034      US-A1- 2019 183 579

- **YOUNG D F ET AL: "Flow characteristics in
models of arterial stenoses - II. Unsteady flow",
JOURNAL OF BIOMECHANICS, PERGAMON
PRESS, NEW YORK, NY, US, vol. 6, no. 5, 1
September 1973 (1973-09-01), pages 547 - 559,
XP026275364, ISSN: 0021-9290, [retrieved on
19730901], DOI: 10.1016/0021-9290(73)90012-2**
- **LYRAS KONSTANTINOS G. ET AL: "An improved
reduced-order model for pressure drop across
arterial stenoses", PLOS ONE, vol. 16, no. 10, 1
October 2021 (2021-10-01), pages e0258047,
XP093053367, DOI: 10.1371/
journal.pone.0258047**

Description

FIELD OF THE INVENTION

The invention belongs to the field of medical diagnostics. The invention is a computer-implemented method for *in silica* evaluation of a human heart condition.

BACKGROUND OF THE INVENTION

Whilst, inherently, every fluid flow is a transient flow, practically this aspect is considered very rarely. Most of the computational problems related to a fluid mechanics usage utilize a steady flow approach which provides enough information about the evolution of a steady flow. Only a small group of practical problems requiring analysis with regard to the whole complexity of the evolution of flow in time existed over decades of studies. These practical problems include a hydraulic shock (water hammer, fluid hammer), flow measurements and a control equipment for use in said measurements including equipment that uses a fluid as a medium transmitting force (for example, an anti-lock braking system (ABS)), tides and a hydraulic wave. These practical problems exist in many fields of technology including aerospace, and automotive. The whole complexity of evolution in time was also studied on a more basic level by two hydraulic engineers - Samuel Grace (for example in Grace SF (1928) Oscillatory motion of viscous liquid in a long straight tube, The London, Edinburgh, and Dublin Philosophical Magazine and Journal of Science, 5(31): 933-939) and Johan Schönfeld. Schönfeld published an article in 1949 (Schönfeld JC (1949) Resistance and inertia of the flow of liquids in a tube or open channel, Flow Turbulence and Combustion, 1(1): 169-197), where he studied a few types of motions of a laminar flow and of a general slow motion in few chosen-by-author cases. According to the article, a total flow and pressure drop relation can be described by the impedance of a channel or a tube. Interestingly, at the beginning of the 20th-century cardiology was another area of application requiring the whole complexity of flow evolution in time due to the development of catheterization techniques.

The history of the evolution of catheterization techniques (including cardiac catheterization techniques) is much longer than that - it goes four hundred years back. William Harvey in 1628 and one hundred years later Stephen Hales made major steps in the description of blood circulation and in the measurement of arterial pressure, respectively. The 19th century was a golden age for the development of understanding of cardiovascular physiology with the most prominent contribution from Claude Bernard, Étienne-Jules, and Carl Ludwig. Cardiac catheterization of humans started in the 20th century and Werner Forssman was the first that successfully performed a heart catheterization on a human being. Werner Forssman carried out this procedure in 1929 on himself. Later in 1940s, André Cournand and Dickinson Richards introduced a diagnostic cardiac catheterization, and in 1960s Mason Sones described a selective coronary angiography. In the 1970s, Andreas Gruentzig initiated catheter-based interventions, which resulted in the expansion of main techniques and in the progress of refinement. Today, the Sones technique is used rarely and a percutaneous coronary intervention and a coronary angiography consist mostly of approaches including percutaneous radial and percutaneous femoral arteries (Bourassa MG (2005), The history of cardiac catheterization, The Canadian Journal of Cardiology, 21(12): 1011-1014).

Propagation of knowledge about benefits, but also risks, related to heart catheterization techniques for functional insufficiency assessment occurred in the late '60s and '70s of the 20th century. This attracted the attention of hydraulic engineers to these techniques. In 1973, two ground-breaking results of works by Donald Young and Frank Tsai were published.

The starting point for building unsteady (transient) flow characteristics of arterial stenoses was a result of theoretical analysis described in the Schönfeld's article

$$\Delta p = \frac{32\eta L}{D^2} v + \frac{4}{3} \varrho L \frac{dv}{dt}, \qquad (1)$$

where: $\Delta p$ - pressure drop, $L$ - distance, $\eta$ - liquid viscosity coefficient, $D$ - tube diameter, $\varrho$ - liquid density, $v$ - mean instantaneous velocity, and $dv/dt$ - rate of changes of mean velocity. The flow characteristics of fluids have an established meaning known for a skilled person and can include any or all from velocity, pressure changes and the Reynolds number as well as other parameters.

The first part of the right-hand side of the equation (1) above describes a pressure drop due to viscosity effects in a steady flow and the second part of the right-hand side is about an additional pressure force necessary for acceleration of a liquid. It was noted in a summary of this article that the value of a highfrequency inertance ($H$) is smaller than the value of a low frequency one ($L$), and that difference between them for a laminar flow is: 20% in a wide open channel or a crevice, 38% in a square tube and 33% in a round tube, and for a turbulent flow it is: 0.25 - 1.5% in a rough or smooth open channel, 0.5 - 3.5% in a crevice or a rough open channel, 0.5 - 3% in a smooth round tube and 1-8% in a rough round tube (in Schönfeld (1949)).

Young and Tsai in 1973 published another article (Young DF, Tsai FY (1973) Flow characteristics in models of arterial stenoses. II. Unsteady flow, Journal of Biomechanics, 6(5): 547-559), where an approximate equation of a transient pressure drop on stenosis was developed and experimentally verified. An experiment was performed on a tube and researchers were investigating effects of stenosis in the steady flow and effects of unsteadiness by using nonsymmetric and axisymmetric models. These two researchers also used a hot-film to investigate turbulences and their development. Young and Tsai proved in this article that for models with (i) a severe constriction a steady flow was marginally more stable than an oscillating flow and for models with (ii) a mild constriction a steady flow was less stable than a corresponding oscillating flow, and that (iii) three (3) parameters with no dimension can characterise an unsteady flow through stenosis. These researchers mentioned an equation from Fry *et al.* (1956, 1959) and from Daily *et al.* (1956). This research by Young and Tsai leads to a pressure drop in the form of

$$\Delta p = K_v \frac{\eta}{D} v + \frac{K_t}{2} \left[ \frac{A_0}{A_1} - 1 \right]^2 \times \varrho |v| v + K_u \varrho L \frac{dv}{dt}, \tag{2}$$

where: $K_v$ - viscous coefficient, $\eta$ - viscosity coefficient, $D$ - tube diameter, $v$ - cross-sectional mean instantaneous velocity in an unobstructed tube, $K_t$ - turbulence coefficient, $A_0$ - cross-sectional area of a tube with no obstacles, $A_1$ - minimal cross-sectional area with stenosis, $\varrho$ - fluid density, $K_u$ - inertial coefficient, $L$ - distance between pressure taps, $dv/dt$ - rate of changes of mean velocity and $|v|v$ is the value of a vector length multiplied by $v$. A steady flow instantaneous mean velocity can be written as $v = v_s + v_n f(t)$, so now the equation above can be written as dimensionless

$$\frac{\Delta p}{\varrho v_p^2} = I_v \bar{v} + I_t \cdot |\bar{v}| \bar{v} + I_u \frac{df}{d\bar{t}}, \tag{3}$$

with:

$$I_v = \frac{K_v}{Re_p}, \ I_t = \frac{K_t}{2} \left[ \frac{A_0}{A_1} - 1 \right]^2, I_u = \frac{K_u L v_n}{\tau v_p^2}, \tag{4}$$

where: $\varrho$ - liquid density, $v_p$ - peak value of cross-sectional mean velocity ($v = v_s + v_n$), $v_s$ - steady component of cross-sectional mean velocity, $v_n$ - amplitude of a periodic component of cross-sectional mean velocity, $K_v$ - viscous coefficient, $Re_p$ - peak Reynolds number ($\varrho v_p D / \eta$), $\bar{v}$ - velocity ratio ($v/v_p$), $v$ - cross-sectional mean instantaneous velocity in an unobstructed tube, $K_t$ - turbulence coefficient, $A_0$ - cross-sectional area of a tube with no obstacles, $A_1$ - minimal cross-sectional area with stenosis, $K_u$ - inertial coefficient, $L$ - distance between pressure taps, $\tau$ - characteristic time, $df/dt$ - function changes in time, $I_v$ - viscous effect index, $I_t$ - turbulence effects index, $I_u$ - inertial effect index. $|\bar{v}|\bar{v}$ is the value of a vector length multiplied by $\bar{v}$.

In 1975 Young *et al.* (Young DF, Cholvin NR, Roth AC (1975) Pressure drop across artificially induced stenoses in the femoral arteries of dogs, Circulation Research, 36(6): 735-743) there is a description of measured *in vivo* pressure losses in artificially narrowed arteries and tests of the usage of equation (2) for forecasting pressure drops. The description discloses an experiment performed on 13 dogs which included inserting an implant into their femoral arteries. A flow rate and a pressure drop on stenosis (severity 52.3 - 92.2%) were measured and mean pressure losses were between 2 and 30 mmHg with peak from 9 to 53 mmHg. This paper also mentions that Young *et al.* were able to forecast pressure losses with deviations of about 20% from the experimental values.

The development of the catheterization method combined with vascular imaging (especially angiography) gave very fast a new context to the Young and Tsai's results. Although, in the '70s of the 20th century there was no knowledge regarding which parameter (index, indicator, etc.) should be used as a criterium of functional assessment.

A scientific article from 1977 by Young *et al.* (Young DF et al. (1977) Hemodynamics of arterial stenoses at elevated flow rates, Circulation Research, 41(1): 99-107) is directly focused on critical stenosis which is a level of stenosis, beyond which blood flow is reduced. The article describes an experiment performed on twelve large mongrel dogs for which stenoses were induced in the range of 55.7 - 91.0 % reduction in carotid and femoral arteries. In this experiment, researchers used hollowed cylindrical plugs, and were measuring pressure drops and instantaneous flow rates - mean velocities were between 3.9 and 88.88 cm/s. An useful index to describe the impact of stenosis is the ratio of maximal flow rate in stenosed vessel ($\overline{Q}_{ms}$) to maximal flow rate with no stenosis ($\overline{Q}_{mo}$). By integrating equation (2) over a flow cycle, Young *et al.* proposed the following equation

$$\overline{\Delta p} = \frac{K_v \eta}{D} \bar{v} + \frac{K_t}{2} \left[\frac{A_0}{A_1} - 1\right]^2 \varrho \overline{|v|v}, \qquad (5)$$

where: $K_v$ - viscous coefficient, $\eta$ - viscosity coefficient, $D$ - tube diameter, $K_t$ - turbulence coefficient, $A_0$ - cross-sectional area of a tube with no obstacles, $A_1$ - minimal cross-sectional area with stenosis, $\varrho$ - liquid density, $\bar{v}$ - time-averaged mean instantaneous velocity over a cycle. Young *et al.* noted that under big pressure loss from distal to stenosis and under vasodilating conditions, a blood flow through a specific artery can be greatly enhanced by a factor between 4 and 5 (in Young et al. (1977)).

Itzchak *et al.* (Itzchak Y et al. (1977) Determination of the pressure drop across an arterial stenosis utilizing angiocinedensitometry, The Yale Journal of Biology and Medicine, 50(4): 375-381) examined 33 healthy patients and 23 patients suffering from intermittent claudication and atherosclerosis obliterans. Severity of intermittent claudication and atherosclerosis obliterans was at the same level. The goal of this medical examination was to present a method of calculating a pressure gradient across stenosis by using flow data derived from angiocinedensitometry. Researchers were calculating a pressure loss across stenotic lesions and the calculations were performed in the left external iliac artery. The Poiseuille flow formula was used to calculate the pressure gradient. The total pressure drop from an inlet into stenosis to any point in a venous system can be expressed as

$$\Delta P = \frac{V_1 \eta 8 L_1}{r_1^2} + \frac{V_2 \eta 8 L_2}{r_2^2}, \qquad (6)$$

where: $V_1$ - flow velocity through a segment, $\eta$ - viscosity, $L_1$ - length of stenosis, $r_1$ - mean radius along a stenotic lesion, $V_2$ - mean flow velocity through a periphery, $L_2$ - theoretical length of the venous system, $r_2$ - theoretical mean diameter of the periphery.

In conclusions, the researchers wrote that in a healthy iliac artery the pressure gradient varies from 0.017 to 0.083 mmHg with mean $0.039 \pm 0.018$ mmHg and the resistance to flow along the artery is $0.076 \pm 0.082$ mmHg min/L. In the atherosclerotic artery the pressure gradient varied from 0.0856 to 3.552 mmHg with mean $0.982 \pm 0.918$ mmHg and the resistance across the stenosis is between 0.26 and 9.6 mmHg•min/L with mean $2.45 \pm 2.4$ mmHg min/L (in Itzchak et al. (1977)).

Gould performed an experiment (Gould KL (1978) Pressure-flow characteristics of coronary stenoses in unsedated dogs at rest and during coronary vasodilation, Circulation Research, 43(2): 242-253) on ten chronically instrumented unsedated dogs to study pressure-flow characteristics. The experiment was performed during rest and involved pharmacological coronary vasodilation on 100 left circumflex stenoses. A Doppler flow transducer was put around the dissected free proximal circumflex artery in order to induce a constriction. A circumferential balloon constrictor was sewn, 2 millimetres distally. A small tapered catheter was placed in the distal main circumflex artery before main branches. Catheters similar to the small tapered catheter were put into the pulmonary artery, root of the aorta and left atrium. According to this experiment, pressure losses by 50% or more caused by an arterial lumen constriction are related to flow velocity and can be described in a simplified form as

$$\Delta P = FV + GV^2, \qquad (7)$$

where: $\Delta P$ - pressure drop in mmHg, $F$ - coefficient of pressure loss due to viscous friction, $V$ - coronary flow velocity in cm/s, $G$ - coefficient of pressure loss due to flow separation or localized turbulence downstream from the stenosis. Simultaneously, Gould proved, that the first and the second part of the right-hand side of the equation (7) described 65% and 35%, respectively, of the total pressure drop at rest, and at peak coronary flow during hyperemia state 33% and 67%, respectively (in Gould (1978)).

Mates *et al.* (Mates RE et al. (1978) Fluid dynamics of coronary artery stenosis, Circulation research, 42(1): 152-162) studied flow through isolated stenotic elements in large coronary arteries. In an experiment, a large-scale coronary circulation model was used to perform a detailed velocity and pressure metering. Servovalves were used to simulate instantaneous peripheral resistance and pulsatile aortic pressure. Researchers examined numerous asymmetric and axisymmetric stenoses and ultimately 4 geometries of stenosis were used in the experiment. A pressure-flow relation in the model can be expressed as

$$\Delta p = \underbrace{\left[\frac{128\eta L}{\pi d_0^{\,4} \int_0^1 \frac{dx}{\left(\frac{d}{d_0}\right)^4}}\right] Q}_{\substack{\text{viscous resistance of} \\ \text{the stenosis}}} + \underbrace{\left[\frac{1.14\varrho}{d_1^{\,4}}\right]}_{\substack{\text{entrance loss} \\ \text{coefficient}}} Q^2 + \underbrace{\left[\left(\frac{2C_d\varrho}{\pi d_0^{\,4}}\right)\left[\left(\frac{d_0}{d_1}\right)^4 - 1\right]\right] Q^2}_{\substack{\text{loss coefficient for} \\ \text{the separated flow}}} + RQ, \qquad (8)$$

where: $L$ - length of stenosis, $Q$ - mean flow, R - peripheral bed resistance, $\eta$ - fluid viscosity, $d_0$ - diameter of tube, $d$ - diameter of canine coronary artery, $d_1$ - minimum equivalent, $\varrho$ - fluid density, $C_d$ - orifice discharge coefficient. In the paper it was admitted that the chosen model was very simple when compared to a complicated coronary circulation model. The chosen model included a straight rigid tube with narrowing elements inserted in series and the elements had resistance of a peripheral bed. It was also noted that recovery of a pressure downstream constriction throat was reduced (or even there was no recovery at all in some cases). A pressure loss was not determined by geometry of stenosis but by its minimal area, and at normal heart rate flow was quasi-steady (in Mates et al. (1978)).

McMahon *et al.* (McMahon MM et al. (1979) Quantitative coronary angiography: measurement of the "critical" stenosis in patients with unstable angina and single-vessel disease without collaterals, Circulation, 60(1): 106-113) were examining 15 patients divided into 2 groups. The first group contained 10 patients with a normal left ventricular (LV) angiogram and a single-vessel coronary disease with no collateralization, ischemic ST-T changes, but no infraction, and a new onset of refractory rest angina. The second group contained 5 patients and was almost like the first group, but these 5 patients had subendocardial infraction (SEI). Researchers were referring to a geometry obtained from a patients' quantitative angiography and they were researching proximal coronary artery critical stenosis. McMahon *et al.* proposed that a pressure drop across a moderately severe narrowing can theoretically be assessed on the basis of the following relation

$$\Delta P = \frac{k_1 Q + k_2 Q^2}{d_{min}^4}, \qquad (9)$$

where: $k_1$ and $k_2$ - constants based on blood viscosity, blood density, lesion shape and length, and flow assumptions, $Q$ - flow, $d_{min}$ - minimal diameter of stenosis. The following results were obtained for the first and the second group, respectively: a minimum stenosis diameter of $0.88\pm0.14$ and $0.64\pm0.08$ mm, a diameter reduction of 72% and 78%, and a cross-sectional area reduction of 92% and 95%. The observed differences between the results for the first and the second group originated from big differences in hemodynamics (in McMahon et al. (1979)).

In an extensive work written by Bessems (Bessems DD (2007) On the propagation of pressure and flow waves through the patient specific arterial system, PhD thesis, TU Eindhoven), in the 4th chapter, the author wrote an overview of a study on a pressure loss across stenosis as a function of local flow characteristics and geometry of stenosis described using the Womersley parameter and the Reynolds number. Bessems in this work used the Young and Tsai's work from 1973. The author was making computations based on a one axisymmetric model of stenosis geometry. The final equation describing the relation between flow characteristics and pressure drop caused by stenosis was expressed as

$$\Delta p_s = K_v(\alpha)R_s q + \frac{\varrho K_t}{2A_0^2}\left(\frac{A_0}{A_s} - 1\right)^2 |q|q + K_u L_u \frac{\partial q}{\partial t} + K_c(\alpha)R_s \bar{q}, \qquad (10)$$

with

$$K_v = 1 + 0.0053 \frac{A_s}{A_0}\alpha^2, K_t = 0.95, K_u = 1.2, K_c = 0.0018\alpha^2, \qquad (11)$$

$$R_s = R_0 \int_0^{Ls} \frac{a_0^4}{a^4(z)} dz, \qquad (12)$$

$$L_u = \frac{\varrho}{A_0} \int_0^{Ls} \frac{a_0^2}{a^2(z)} dz, \qquad (13)$$

where: $K_v$ - viscous coefficient, $\alpha$ - Womersley parameter, $R_s$ - friction coefficient, $q$ - flow, $\varrho$ - blood density, $K_t$ - turbulence coefficient, $A_0$ - cross-sectional area of the lumen vessel proximal to the stenosis, $A_s$ - cross-sectional area of the lumen vessel at the neck of stenosis, $K_u$ - unsteadiness coefficient, $L_u$ - equation (18) coefficient, $t$ - time, $K_c$ - offset, $a_0$ - radius of the lumen vessel proximal to the stenosis, $a$ - stenosis geometry, $L_s$ - stenosis length, $R_0$ - is representing the coefficient of friction for steady flow in a straight tube per unit of length, $z$ - axial position. Author compared a pressure drop from a numerical simulation to the one based on an equation from Young and Tsai's work, and a pressure loss from the final equation to a numerical result - the differences were up to 80% and 10%, respectively (in Bessems (2007)). Interestingly, a pressure loss transient component is again referred to in the work by Bessems (2007).

An analogous concept is shown in Liang *et al.* (Liang F et al. (2009) Multi-scale modelling of the human cardiovascular system with applications to aortic valvular and arterial stenoses, Medical & Biological Engineering & Computing, 47(7): 743-755) and this concept was created on the basis of multi-scale modelling including an entire cardiovascular system computational model. The fifty-five largest arteries that are making arterial tree, were modelled in 1-D and their geometry was described based on data published in works by Stergiopulos *et al.* and Olufsen. Assuming that a pressure-flow relationship across a normal cardiac valve is consistent with the Bernoulli law and considering a contribution of blood inertia and viscous resistance, a pressure drop equation can be expressed as

$$\Delta P_{cv} = R_{cv}Q_{cv} + B_{cv}Q_{cv}|Q_{cv}| + L_{cv}\dot{Q}_{cv}, \qquad (14)$$

where: $R_{cv}$ - coefficient of viscous, $Q$ - flow rate through stenosis, $B_{cv}$ - coefficient of flow separation, $L_{cv}$ - coefficient of inertial terms, $\dot{Q}$ - is the time derivative of $Q$.

But when it comes to severe aortic valvular (AV) stenosis, energy loss related to a sudden expansion of flow from vena contracta to the ascending aorta dominates in transvalvular pressure loss. Researchers took the model proposed by Garcia *et al.*

$$\Delta P_{av} = \frac{1}{2}\varrho Q_{av}^2 \left(\frac{1}{EOA} - \frac{1}{A_{ao}}\right)^2 + 2\pi\varrho \frac{\partial Q_{av}}{\partial t}\left(\frac{1}{EOA} - \frac{1}{A_{ao}}\right)^{0.5}, \qquad (15)$$

where: $\varrho$ - blood density, $Q_{av}$ - transvalvular flow rate, $EOA$ - effective orifice area, $A_{ao}$ - cross-sectional area of the ascending aorta, $t$ - time. In conclusions, the researchers mentioned that stenoses localization are a crucial factor of a global hemodynamic impact of an arterial constriction in an arterial system. The equation (15) shows similarities to a previous assessment models of a pressure drop on stenosis under transient conditions, but it is dedicated only to a local assessment of a pressure drop, and it cannot be applied directly on across the whole length of a vessel (in Liang et al. (2009)).

An attempt of a direct diagnostic usage of trans-stenotic pressure loss assessment models initiated by Donald Young and Frank Tsai is provided by Huo *et al.* (Huo Y et al. (2012) A validated predictive model of coronary fractional flow reserve, Journal of the Royal Society, Interface, 9(71): 1325-1338). These researchers derived a pressure drop and myocardial FFR model using physics for a coronary artery constriction. To verify the model, researchers performed *in vivo* (in arteries of 8 swine an occluder cuff was used to induce narrowing) and *in vitro* (in isolated arteries with asymmetric and symmetric tubes, where an inflatable occluder cuff was used to induce narrowing) experiments. A proposed analytical model uses a general Bernoulli equation

$$\Delta P = \Delta P_{convective} + \Delta P_{constriction} + \Delta P_{diffusive} + \Delta P_{expansion}, \qquad (16)$$

where: $\Delta P_{convective}$ - energy loss as a consequence of flow convection, $\Delta P_{constriction}$ - energy loss due to sudden narrowing in cross-sectional area (CSA) from a normal proximal vessel to a stenosis, $\Delta P_{diffusive}$ - energy loss due to flow diffusion, $\Delta P_{expansion}$ - energy loss due to a rapid expansion in cross-sectional area (CSA) from a narrowing to a normal distal vessel. The total pressure loss on a stenosis can be written in two ways, first as

$$\Delta P_s = \frac{\varrho Q^2}{2CSA_s^2} \times \frac{96}{5} \int_\alpha^1 \frac{(1 + 4\alpha + 9\alpha^2 + 4\alpha^3)}{\alpha(3 + 2\alpha)(3 + 2\alpha + \alpha^2)^2} d\alpha + \Delta P_{expansion}^{blunt} \qquad (17)$$

when $\alpha \geq 0.05$ for $(L_s/Re_s) \cdot D_s$, and second as

$$\Delta P_s = \frac{\varrho Q^2}{2CSA_s^2} \times \frac{96}{5} \int_{0.05}^{1} \frac{(1 + 4\alpha + 9\alpha^2 + 4\alpha^3)}{\alpha(3 + 2\alpha)(3 + 2\alpha + \alpha^2)^2} d\alpha + \int_{0}^{L_s - L_e} \frac{8\pi\mu}{CSA_s^2} Q dx + \Delta P_{expansion}^{parabolic} \qquad (18)$$

when $\alpha < 0.05$ for $(L_s/Re_s) \cdot D_s$. The following symbols are used in equations: $\varrho$ - blood density, $Q$ - flow rate, $CSA_s$ - stenotic cross-sectional area, $\alpha$ - radius of inviscid core (dimensionless), $\Delta P_{expansion}^{blunt}$ - pressure loss caused by a sudden expansion in CSA for blunt flow velocity profile at a stenosis outlet, $L_s$ - stenosis length, $L_e$ - length of entrance for $\alpha$=0.05, $\eta$ - dynamic viscosity, $x$ - position, $\Delta P_{expansion}^{parabolic}$ pressure loss caused by sudden expansion in CSA for parabolic flow velocity profile at stenosis outlet, $Re_s$ - Reynolds number at a stenosis inlet, $D_s$ - inlet diameter of a stenosis. Huo *et al.* confirmed that the model proposed by researchers agreed with measurements from experiments and also concluded that their coronary stenosis analytical model enables predicting FFR on the basis of a hyperaemic coronary flow and a stenosis dimension without any empirical parameters (in Huo et al. (2012)).

In an article written by Itu *et al.* (Itu L et al. (2013) Non-invasive hemodynamic assessment of aortic coarctation: validation with in vivo measurements, Annals of Biomedical Engineering, 41(4): 669-681), the assessment based on CFD combined with an innovative, non-invasive model personalization approach for a non-invasive hemodynamic assessment of post- and pre-operative patients with coarctation of the aorta (CoA) was suggested. The researchers proposed a comprehensive pressure drop for coarctation

$$\Delta P = \underbrace{K_V(\alpha) \cdot R_{VC} q}_{\text{viscous losses}} + \underbrace{\frac{\varrho K_t}{2A_0^2} \left(\frac{A_0}{A_c} - 1\right)^2 |q|q}_{\text{turbulent losses}} + \underbrace{K_u L_u \frac{\partial q}{\partial t}}_{\substack{\text{inertial} \\ \text{effect}}} + \underbrace{K_c(\alpha) R_{VC} \bar{q}}_{\substack{\text{continous} \\ \text{component}}}, \qquad (19)$$

with

$$K_v = 1 + 0.053 \left(\frac{A_c}{A_0}\right) \alpha^2, K_t = 1.52, K_u = 1.2, K_c = 0.0018\alpha^2, \qquad (20)$$

$$R_{vc} = \frac{8\mu}{\pi} \int_{0}^{L_c} \frac{1}{r^4(l)} dl, \qquad (21)$$

$$L_u = \frac{\rho}{\pi} \int_{0}^{L_c} \frac{1}{r^2(l)} dl, \qquad (22)$$

where: $K_v$ - coefficient of viscosity, $\alpha$ - Womersley number, $R_{vc}$ - viscous resistance, $q$ - flow, $\rho$ - liquid density, $K_t$ - turbulence coefficient, $A_0$ - normal cross-sectional area, $A_c$ - minimum cross-sectional area of coarctation, $K_u$ - coefficient of inertance, $L_u$ - length of coarctation, $t$ - time, $K_c$ - continuous coefficient, $\bar{q}$ - average flow, $L_c$ - length of coarctation, $\mu$ - dynamic viscosity of fluid.

In preliminary results Itu *et al.* received an absolute error smaller than 2 mmHg. To calculate centrelines of arteries and different radius measurements, a vessel tree segmented 3-D geometric model was used. To obtain the lumen of vessel tree segmentation, researchers used 3-D contrast enhanced MR angiograms (MRA) (in Itu et al. (2013)).

A beautiful and a practical summary of these studies including Bessems (2007) and Itu L *et al.* (2013) can be found in specifications of patents obtained by Sharma *et al.* (2013) Non-invasive functional assessment of coronary artery stenosis including simulation of hyperemia by changing resting microvascular resistance, U.S. Patent No. 10373700 B2 and Sharma *et al.* (2013) Non-invasive functional assessment of coronary artery stenosis including simulation of hyperemia by changing resting microvascular resistance, U.S. Patent No. 10354744 B2. US 2019/0183579 A1 describes patient-specific modeling of hemodynamic parameters in coronary arteries.

In clinical practice a functional assessment of a circulatory insufficiency is realized following two alternative strategies: (1) magnitude of pressure changes due to flow assessment, or (2) variation of blood flow supply at certain pressure conditions. Because escalation of circulatory pathology symptoms occurs often in stress conditions and not in rest, the assessment is made in stress conditions (usually achieved by pharmacological means). According to the current standards (Lawton JS et al. (2022) 2021 ACC/AHA/SCAI Guideline for Coronary Artery Revascularization: Executive Summary: A Report of the American College of Cardiology/American Heart Association Joint Committee on Clinical Practice Guidelines, Circulation, 145(3): e4-e17; Knuuti J et al. (2020) 2019 ESC Guidelines for the diagnosis and management of chronic coronary syndromes, European heart journal, 41(3): 407-477) a recommended strategy is the first one which is the one based on magnitude of pressure changes.

It is recommended to perform assessment using a coronary reserve ratio including FFR, iFR or equivalent (for example, the ESC Guidelines also mention FFR-CT obtained *in silico*). The ratio can be calculated as a ratio of mean of a distal pressure and a proximal pressure: $FFR = P_d/P_a$, so $FFR = 1 - \Delta P/P_a$. This is because the distal pressure ($P_d$) at a given cross-section can be expressed as a difference of a proximal pressure (for example, a pressure at a coronary artery inlet ($P_a$) cross-section) and a pressure loss on a flow path between both cross-sections ($\Delta P$). The capital letter $P$ represents a mean pressure value, and the lowercase letter $p$ represents its instantaneous value (so $P = (1/T) \cdot \int_T p \, dt$, where $T$ is time interval of averaging). Methods relating to a flow assessment, so a blood supply deficit, even if they are being used, currently they are not the gold standard in diagnostics.

In *in vivo* conditions, an FFR measurement procedure is based on two pressure measurements ($P_a$ and $P_d$) and both are using a pressure wire which is placed inside the artery. Even though the procedure is not time consuming, its realization can be complicated and the procedure may be harmful for a patient. Placing the pressure wire in the artery involves danger. Said danger is not introduced by *in silico* methods (including CFD - computational fluid dynamics) which makes *in silico* methods a very important and prospective alternative for *in vivo* methods. Unquestionably, computational fluid dynamics - although with difficulty, but successfully - gets increasing clinicians' trust in diagnostics usage (Cook CM et al. (2017) Diagnostic accuracy of computed tomography-derived fractional flow reserve: A systematic review, JAMA cardiology, 2(7): 803-810; Driessen RS et al. (2019) Comparison of coronary computed tomography angiography, fractional flow reserve, and perfusion imaging for ischemia diagnosis, Journal of the American College of Cardiology, 73(2): 161-173; Torii R, Yacoub MH (2021) CT-based fractional flow reserve: development and expanded application, Global cardiology science & practice, E202120; Pontone G et al. (2022) Clinical applications of cardiac computed tomography: a consensus paper of the European Association of Cardiovascular Imaging-part II, European heart journal, Cardiovascular Imaging, 23(4): e136-e161). Especially a coronary CT angiography-derived fractional flow reserve gains followers.

A user of *in silico* diagnostic tools expects achieving the highest accuracy, sensitivity, specificity and so on. Naturally this involves striving for the most precisely mapping of geometry and also using a computation mesh with an extremely high mesh density. Even if this one-way strategy allows to achieve an increase in accuracy, it does not consider practical aspect of feasibility and usability of this type of tools in diagnostics. This is one of the aims of the invention. In fact, increasing a computing mesh density is always associated with an exponential increase of computational effort. In addition, numerical CFD algorithm stability criteria, especially a Courant-Friedrichs-Lewy (CFL) convergence condition (Courant R, Friedrichs K, Lewy H (1928) Über die partiellen Differenzengleichungen der mathematischen Physik, Mathematische Annalen, 100, 32-74; de Moura CA, Kubrusly CS (2013) The Courant-Friedrichs-Lewy (CFL) condition: 80 years after its discovery, Birkhauser Boston) imposes a requirement

$$Co = \frac{v\Delta t}{\Delta h} \le C_{max}, \qquad\qquad (23)$$

where: v - velocity, $\Delta h$ - mesh density factor, $\Delta t$ - time step length. Existence of these criteria leads to a paradoxical situation in which $\Delta h \to 0$ and $\Delta t \to 0$ and to instability of calculations.

In this paradoxical situation not only the computational effort for one time step $\Delta t$ increases exponentially with an increase of a number of mesh nodes, but also concurrently there is a dramatic increase in a number of time steps ($\Delta t$). It can be roughly estimated that few thousands or even few millions of time steps are needed for one cardiac cycle coronary flow simulation. From a clinician's standpoint, one cardiac cycle coronary flow simulation requires enormous time to be completed whereas a standard *in vivo* procedure is completed typically from 10-15 minutes up to an hour. In addition, an on-demand availability of high computing power is required to maintain the needed availability of *in silico* diagnostic services. Today's providers of these diagnostic services ensure availability of *in silico* diagnostic services using cloud computing technologies such as Microsoft Azure or Amazon Web Services. It is expected that once said *in silico* diagnostic services become routinely used on a global scale, there will not be enough resources available to ensure the needed on-demand availability without improving performance of *in silico* simulation. What also remains a factor is that high computing power requires a large amount of electrical energy. This large amount of electrical energy has a negative impact on the environment (carbon footprint).

There is a need for an accurate *in silico* method of diagnostic assessment of a human heart hemodynamic condition which is available on-demand and does not require a large computational effort to be realized.

**BRIEF DESCRIPTION OF THE INVENTION**

The forthcoming brief description is to better understand the principle and benefits of the invention claimed in the appended claims. Therefore, it is not meant to be limiting in any sense.

The invention is a computer-implemented method for calculation of a pressure drop ($\Delta p$) between a first cross-section and a second cross-section of an artery. In embodiments, artery can be a coronary artery, carotid artery, renal artery, peripheral artery, or aorta. According to the invention the second cross-section is downstream from the first cross-section and the two sections are separated by a channel which has a length of $l$. As it can be understood by a skilled person, the length of the channel is a distance between the two cross-sections. Depending on the context, this disclosure is using both distance and length to describe aspects of the invention. The channel is a part of the artery and, if not obstructed, allows blood (or any other fluid) to flow from the first cross-section to the second cross-section. Term "downstream" refers to a direction of flow of blood in a healthy human being. For example, a point in an artery downstream of an inlet to the artery is the point towards which blood is flowing from the inlet.

The method comprises steady-state computational fluid dynamics (CFD) simulations but allows to calculate the pressure drop ($\Delta p$) and, what is more, a transient pressure drop. This is done by separating the pressure drop into a steady-state pressure loss component and into a transient pressure loss component.

The invention takes a different approach to the one known from methods of assisting diagnosis based on transient flow CFD simulations (such as vFFR, CT-FFR, FFR-CT, etc.). The invention formulates its simulations in a completely different way with respect to those methods by avoiding using of transient CFD simulations to directly calculate transient flows. Instead, the invention implements steady-state CFD simulations to reach the transient flows. This allows achieving hundreds or even thousands of times faster simulations speed while at the same time maintaining the required level of accuracy for diagnostics. This allows obtaining faster diagnosis and treatment. In certain embodiments aiming for a practical implementation of the invention, the invention is focused on the pressure loss assessment.

In an aspect of the invention, the steady-state pressure loss component is formulated such that it allows for reliable and fast reaching a diagnosis.

A distal pressure $p_d$ is a function of a current flow $q$, and is described as a proximal pressure $p_a$ reduced by a transient pressure drop $\Delta p$ over a flow path $l$ between distal and proximal locations, i.e.

$$p_d = p_a - \Delta p. \qquad (24)$$

Obtained measured instantaneous pressure values are averaged to calculate a FFR index. The values obtained from computations of a FFR index are instantaneous. For computations, the values of a distal pressure, a proximal pressure and a current flow in a cross-section can be derived using patient-specific variables, as done in: Kosior A, Mirota K, Tarnawski W (2019) Patient-specific modelling of hemodynamic parameters in coronary arteries (WO 2020/048642 A1, or in EP 3820357 B1). Values of distal pressure can also be obtained from non-invasive measurements.

According to the invention, the pressure drop ($\Delta p$) can be expressed as

$$\Delta p = \Delta p_s + \Delta p_t \qquad (25)$$

with $\Delta p_s$ being a steady-state pressure loss component and $\Delta p_t$ being a transient pressure loss component. Various embodiments described in the detailed description show how to calculate the steady-state pressure loss component and the transient pressure loss component. Unless stated otherwise, all embodiments and elements of said embodiments can be combined together to form new embodiments that are within the scope of this disclosure.

Main application of the invention is in a trans-stenotic pressure drop assessment and, associated with this assessment, in arriving at a diagnostic index, especially Fractional Flow Reserve (FFR) through computer simulations. The diagnostic index is relevant, for example, for diagnosis and treatment of medical conditions related to a human heart.

In an embodiment, the pressure drop is calculated using only the steady-state pressure loss component

$$\Delta p = \Delta p_s \qquad (26)$$

The disclosure is providing various ways to calculate the steady-state pressure loss component. According to an embodiment, the steady-state pressure loss component is expressed as

$$\Delta p_s = c_0 + c_1 \cdot q + c_2 \cdot |q| \cdot q \qquad (27)$$

where $c_0, c_1, c_2$ are empirical coefficients which can be calculated using steady-state computational fluid dynamics (CFD) simulations. This embodiment allows to calculate, for example, FFR using steady-state CFD simulations. Benefits of this embodiment are pace of calculations needed to obtain the FFR index and that is relevant for diagnosis and treatment.

In another embodiment, the steady-state pressure loss component is expressed as

$$\Delta p_s = \Delta p_0 + \Delta p_1 + \Delta p_2 \tag{28}$$

where $\Delta p_0$ is zero flow pressure, $\Delta p_1$ is viscous resistance for straight parts of the channel or parts of the channel with radius of curvature ($R$) to radius of the part ($r$) equal $r/R < 10$, and $\Delta p_2$ is local resistance for parts of the channel with a stenosis. The radius of curvature ($R$) to radius of the part ($r$) equal $r/R < 10$ ensures that the channel or its parts are relatively straight. In embodiments, $r/R$ can be below 9, 8, 7 or 6. Stenosis in the channel of the artery has an established meaning known to the skilled person. Stenosis can be understood as narrowing or blockage of an artery.

In an embodiment of the invention, the viscous resistance ($\Delta p_1$) is equal to

$$\Delta p_1 = f \frac{l}{\langle d \rangle} \varrho \frac{\langle v \rangle^2}{2} \tag{29}$$

where $f$ is hemodynamic friction coefficient, $\varrho$ is density of blood. $\langle d \rangle$ is "equivalent" inner diameter of the channel. $\langle d \rangle$ is not measured but it is calculated for a model of the channel which has a round cross-section and length identical to the length of the channel for which the model is created. $\langle d \rangle$ is calculated assuming that pressure loss for a laminar flow is the same for the channel and for the model of the channel. $\langle v \rangle$ is "equivalent" flow velocity and is also calculated for the model of the channel while assuming that pressure loss for a laminar flow is the same for the channel and for the model of the channel.

The local resistance ($\Delta p_2$) is proportional to $\varrho \langle v \rangle^2/2$. Calculating of the pressure drop using $\Delta p_1$ and $\Delta p_2$ allows to reliably reproduce results obtained in clinical trials while not requiring large computing power.

The "equivalent" inner diameter and the "equivalent" flow velocity are terms used in the field of the invention and have an established meaning therein. This meaning should not be confused with equivalents used in law.

In embodiments, the pressure drop ($\Delta p$) includes a transient pressure loss component and has a from of

$$\Delta p = \Delta p_s + \Delta p_t. \tag{30}$$

In these embodiments, the transient pressure loss component ($\Delta p_t$) can be computed in accordance with the Isaac Newton's second law of motion. More specifically, the transient pressure loss component *(Δpt)* can be expressed as

$$\Delta p_t = k_t \frac{\varrho l}{< A_0 >} \frac{dq}{dt} \tag{31}$$

where $t$ is time and $k_t$ is empirical index defined within the range 1 and 1.2. In embodiments, $k_t$ can be equal to 1.6, 1.7, 1.8, 1.85, 1.9, 1.95, 1.96, 1.97 or 1.98. $<A_0>$ is "equivalent" surface area which is calculated and not measured. $<A_0>$ is calculated using $\langle d \rangle$ as, for example, $<A_0> = (\pi/4)\langle d \rangle^4$. In analogous way, the skilled person can calculate $\langle v \rangle$ using $<A_0>$. The pressure drop $\Delta p$ including calculation of the steady-state pressure loss component and the transient pressure loss component *(Δpt)* is the above-mentioned transient pressure drop. The transient pressure drop is reconstructed based on a proposed relationship describing flow characteristics in which empirical parameters are selected using the results of steady-state series of CFD simulations. This aspect of the invention allows to eliminate complex and difficult transient CFD simulations, thereby saves time needed to computations and also avoids the need to use of high computer power. This allows the invention to be available on-demand and decreases any environmental impact of *in silico* diagnostic methods.

In embodiments, the pressure drop $\Delta p$ is defined between two cross-sections of a coronary artery. In more specific embodiments, the pressure drop $\Delta p$ is defined between an inlet to the coronary artery and a point in the coronary artery downstream the inlet to the coronary artery.

In more specific embodiments, the two cross-sections and/or the channel between the two cross-sections have identical or similar topology. Similar topology is defined using a similarity index which takes into account change in internal diameter between the two cross-sections and/or across the artery between the two cross-sections, branching at the two cross-sections and/or across the artery between the two cross-sections or all of the above. The similarity index can have a value of 50%, 55%, 60%, 65%, 70%, 75%, 80%, 85%, 90%, 95% or 99% in embodiments of the invention. The similarity index can be defined as a range created using any of the above-mentioned values. The similarity index can be defined as above 50%, above 55% etc.

Various details and other embodiments of the invention will be presented in the forthcoming description.

## BRIEF DESCRIPTION OF DRAWINGS

The invention will now be described in more detail with reference to the appended figures in which: Fig. 1 presents results of Coronary Computed Tomography Angiography (CCTA) and visualization of features of the invention used in obtaining fully transient pressure drop in a coronary aorta, Fig. 2 includes a diagram of an embodiment of the invention, Fig. 3 presents an example of finding "equivalent" diameter for a patient according to the invention, Fig. 4 presents computations of the steady-state pressure loss including the Lagrange and Reynolds numbers according to the invention, Fig. 5 shows a comparison of the results of measurements of the fractional flow reserve for 1960 *in vivo* human cardiac cycles, calculated on the basis of the approximation of the steady-state pressure loss component, and Fig. 6 contains a comparison of transient pressure drop from medical data with the one reconstructed by using the invention.

## DETAILED DESCRIPTION

In an embodiment of the disclosure, the established relationship describing the pressure drop $\Delta p$ between two cross-sections of an artery is equal to:

$$\Delta p = \Delta p_s + \Delta p_t = c_0 + c_1 \cdot q + c_2 \cdot |q| \cdot q + \Delta p_t, \tag{32}$$

where $c_0$, $c_1$, $c_2$ are empirical coefficients and $q$ is a flow. Values of the empirical coefficients can be calculated using the results of a series of steady-state CFD simulations thereby enabling computation of a pressure loss $\Delta p_s$ using steady-state simulations. Values of the empirical coefficients can be calculated using the results of clinical trials or using a combination of clinical trials and steady-state CFD simulations. This makes the pressure loss $\Delta p_s$ a steady-state component. In embodiments, only $\Delta p_s$ can be calculated (without $\Delta p_t$) which allows to obtain, for example, FFR. In more preferable embodiments, $q$ is within the range of 1 ml/s to 15 ml/s. This ensures higher accuracy level of results. Even more preferably, $q$ is equal to 1.5 ml/s, 3.5 ml/s or 5 ml/s. Alternatively, the most precise results can be obtained following these steps: (1) approximating typical $q_0$ value for a given artery using, for example, Milnor WR (1982) Normal hemodynamic state, in Hemodynamics, Baltimore, Williams&Wilkins, (2) establishing a range from $q_0/5$ to $3q_0$, and (3) performing calculations within this range.

The second component $\Delta p_t$ describes an inertial effect which can occur at unsteady flow conditions. This makes $\Delta p_t$ a transient component. This component is not calculated using the results of the steady-state CFD simulations but computed in accordance with the Isaac Newton's second law of motion

$$\Delta p_t = c_3 \frac{dq}{dt} = k_t \varrho l \frac{dv}{dt} = k_t \frac{\varrho l}{A} \frac{dq}{dt}, \tag{33}$$

so, as a quotient of force caused by acceleration/deceleration $dv/dt = (1/A) \cdot (dq/dt)$ of mass $\varrho lA$ between two cross-sections (for example, between proximal and distal cross-sections) where $l$ is distance and $A$ is surface area of artery cross-section. $k_t$ is an empirical index (typically $k_t = 1$) and, in a preferred embodiment, $k_t = 1.2$ which means that the inertial force is increased by 20% with respect to a theoretical value. In embodiments, this empirical index can have different values and include, for example, 0.7, 0.8, 0.9, 1.1, 1.15, 1.16, 1.17, 1.18, 1.19, 1.21, 1.22, 1.23, 1.24, 1.25, 1.3 or 1.35.

The way to determine the transient component $\Delta p_t$ for the invention is fixed and given above in equation (33), and the steady-state component $\Delta p_s$ can be determined using any of many possibilities envisaged for the invention.

As can be seen from equation (33), this embodiment does not utilize transient CFD calculations and only steady-state CFD calculations to reach the transient pressure drop. This provides several orders of magnitude increase in pace of calculating the pressure drop $\Delta p$.

In an embodiment of the invention, the pressure loss $\Delta p_s$ at the steady state is represented by a superposition of a sum of three physical components

$$\Delta p_s = \Delta p_0 + \Delta p_1 + \Delta p_2. \tag{34}$$

The component $\Delta p_0$ is specific for coronary circulation simulations and it corresponds to zero flow pressure (Hoffman JIE (1990) Pressure-flow relationships of the coronary arteries, in: Kajiya F, Klassen GA, Spaan JAE, Hoffman JIE (eds) Coronary Circulation, Springer-Verlag Tokyo, 109-125). Generally, its value is so small that its contribution to the total value of $\Delta p$ (and also $\Delta P$) is marginal. For a healthy person, the contribution is around $14\pm7$ mmHg and with a coronary intervention with only a ST-segment elevation myocardial infarction, it can achieve or even exceed 42 mmHg (Patel N et al. (2015) The zero-flow pressure measured immediately after a primary percutaneous coronary intervention for a ST-segment elevation myocardial infarction provides the most useful invasive index for predicting the extent of myocardial infarction after 6 months as showed in An OxAMI Study (Oxford Acute Myocardial Infarction), JACC, Cardiovascular interventions, 8(11): 1410-1421). In one embodiment, $\Delta p_0$ is a constant value.

The two remaining components $\Delta p_1$ and $\Delta p_2$ vary significantly with flow. $\Delta p_1$ and $\Delta p_2$ are components of a viscous resistance in straight sections (or sections with relatively small curvature - those with radius of curvature ($R$) to radius of section of a vessel ($r$) equal $r/R < 10$, in view of Freidoonimehr et al. (2021) Effect of artery curvature on the coronary fractional flow reserve, Physics of Fluids, 33, 031906) and a local resistance (located in areas with a narrowing or a blockage of artery, for example, at a stenosis), respectively. Irrespectively of the used approach (experiment or simulation) the skilled person recognizes that in embodiments including $\Delta p_1$ and $\Delta p_2$ the values of $\Delta p_1$ and $\Delta p_2$ are the most relevant for accuracy of functional evaluation of a pressure loss (up to 98% as showed by clinical trials). For example, at a stenosis.

In one embodiment of the invention, the viscous resistance component $\Delta p_1$ is approximated according to the Henry Darcy's law, so in a form (Bird RB, Stewart WE, Lightfoot EN (2007) Transport phenomena, John Wiley & Sons; White FM (1999) Fluid Mechanics, Boston Mass: WCB/McGraw-Hill)

$$\Delta p_1 = f \frac{l}{\langle d \rangle} \varrho \frac{\langle v \rangle^2}{2}, \tag{35}$$

where: $f$ - hemodynamic friction coefficient, $l$ - distance from a first cross-section (where proximal pressure $p_a$ is measured, for example from an inlet) to a second cross-section (where distal $p_d$ pressure is to be calculated), $\varrho$ - density of flow medium (here blood) and $\langle d \rangle$ - "equivalent" inner diameter of a channel of coronary artery calculated for a model of coronary artery channel which has a round cross-section and length identical to the length of a real coronary artery channel for which the model is created and while assuming that pressure loss for a laminar flow is the same for the real coronary artery channel and for the model of coronary artery channel. $\langle v \rangle$ is "equivalent" flow velocity calculated for a model of coronary artery channel which has a round cross-section and length identical to the length of a real coronary artery channel for which the model is created and while assuming that pressure loss for a laminar flow is the same as for the real coronary artery channel and for the model of coronary artery channel. The skilled person will appreciate that this approach gives reliable values for $\langle v \rangle$ and $\langle d \rangle$ since, in case of tomography measurements, the resolution of tomography is too low to provide precise values of flow velocity and inner diameter for arteries (0.5 mm resolution where d can be at the level of 4 mm). This embodiment has the same benefits as the embodiment including equation (34) which includes avoidance of transient CFD simulations.

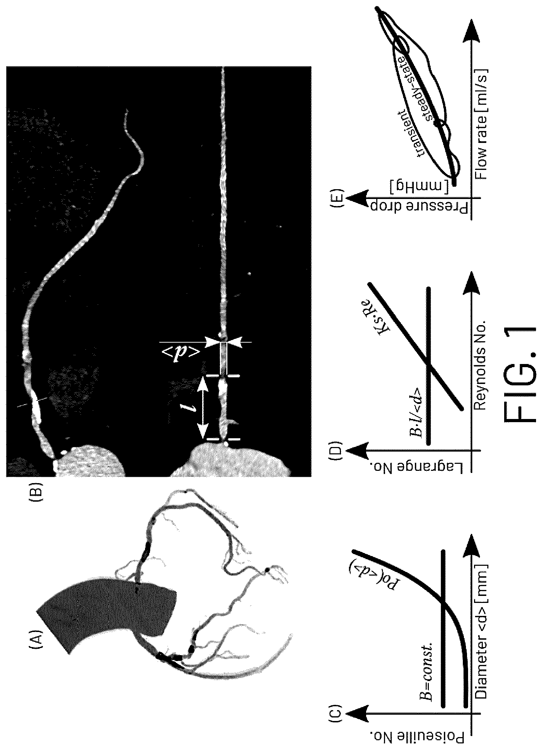

Visualization of an embodiment using $\langle d \rangle$ on CCTA results is shown on Fig. 1. On Fig. 1 (A) there is an aorta and coronary artery tree obtained through CCTA, and on Fig. 1 (B) there is a right coronary artery view and its curved planar reformation (CPR). On Fig. 1 (C), (D) and (E), there are reconstruction principles of "equivalent" diameter $\langle d \rangle$, invariants used to obtain transient pressure drop according to the invention as well as a relation between steady-state and transient pressure drop, respectively.

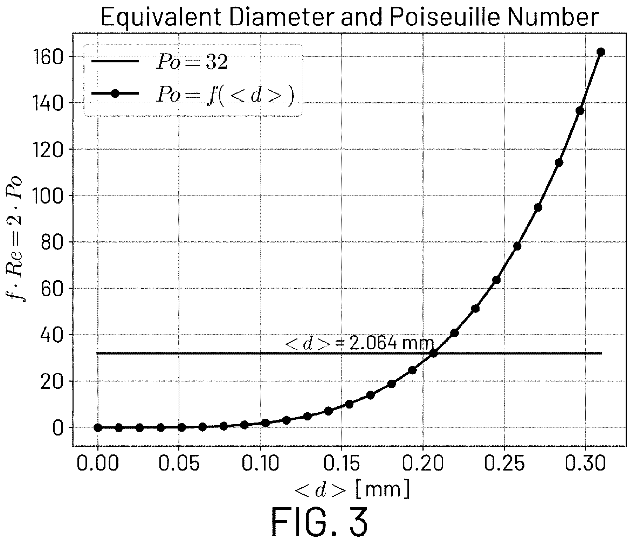

In one embodiment presented on Fig. 3, the value of $\langle d \rangle$ is calculated using equation (53) presented below and the value of $\langle v \rangle$ is calculated using $\langle d \rangle$. Fig. 3 presents an example of finding "equivalent" diameter for a patient using the Poiseuille number. This patient is a 67-year old male (height: 176 cm, weight: 92 kg, BMI: 29.7, systolic and diastolic blood pressures: 145 mmHg and 85 mmHg, respectively, heart rate: 72/min). On Fig. 3, a curve with dots describes changes in the Poiseuille number vs. "equivalent" diameter, and a solid curve presents "equivalent" diameter for the Poiseuille number equal to 32 (laminar flow). It is one of ways of finding "equivalent" diameter for a patient.

$\langle d \rangle$, $\langle v \rangle$ can be, in one embodiment, medium values of an inner diameter of a channel and flow velocity through the channel. In different embodiments, the values of $\langle v \rangle$ and $\langle d \rangle$ are selected such that, for example, mass flow, volume, momentum or energy parameters are corresponding to the parameters obtained for flow velocity and inner diameter obtained from measurements (for example, from tomography measurements).

In one embodiment of the invention, (35) is transformed to a general dimensionless form

$$\frac{\Delta p_1}{\varrho \langle v \rangle^2} = \frac{f}{2} \frac{l}{\langle d \rangle}. \tag{36}$$

Taking into consideration that - in general - the left-hand side expression defines the similarity invariant in the form of Euler number $Eu_1 = \Delta p_1 / (\varrho \cdot \langle v \rangle^2)$, we have ultimately

$$Eu_1 = \frac{f}{2} \frac{l}{\langle d \rangle}. \tag{37}$$

This allows for performing calculations using linear equations instead of quadratic equations which offers significant improvement in terms of pace of calculations. This also allows to decrease the need for high computing power as explained earlier.

In yet another embodiment, in a wide range of physiological conditions, blood flow in straight sections of coronary artery, so except from areas with plaque location, has a laminar character. This is because Reynolds number $Re = \langle v \rangle \cdot \langle d \rangle \cdot \varrho / \eta$, where $\eta$ means viscosity coefficient, is rarely bigger than several hundreds. As a consequence, it is assumed that a hydrodynamic friction coefficient is determined according to the Hagen-Poiseuille's law for the channel of circular cross-section

$$f = \frac{64}{Re}, \tag{38}$$

where $Re$ is the similarity invariant in the form of the Reynolds number

$$Re = \frac{\langle v \rangle \langle d \rangle \varrho}{\eta} = \frac{4}{\pi} \frac{q\varrho}{\langle d \rangle \eta}. \tag{39}$$

In a preferred embodiment, we can also assume the Euler number for loss component in straight sections as

$$Eu_1 = \frac{B}{Re} \frac{l}{\langle d \rangle}. \tag{40}$$

In an embodiment, B is equal to 32.

In an embodiment, the third component ($\Delta p_2$) of pressure loss is a local loss and has a value proportional to dynamic pressure

$$\Delta p_2 \sim \varrho \frac{\langle v \rangle^2}{2}. \tag{41}$$

In a preferred embodiment, the Euler number is expressed as

$$\frac{\Delta p_2}{\varrho \langle v \rangle^2} = Eu_2 \sim const, \tag{42}$$

which means that the Euler number is proportional to a certain constant. If following approximation of a trans-stenotic Young-Tsai loss, Bessems (2007), Itu (2012) and other researchers mentioned earlier, they combine this certain constant with a component having a general form ($< A_0 >/A_s$ - 1)$^2$, where $<A_0>$ and $A_s$ are cross-sectional area of the lumen, respectively, of a vessel based on "equivalent" diameter proximal to the stenosis and at the neck of the stenosis. By taking into consideration that the effect of throttling the flow with atherosclerotic plaque is described in medical literature with a level of stenosis we arrive at (Carnicelli AP et al. (2013) Cross-sectional area for the calculation of carotid artery stenosis on computed tomographic angiography, Journal of vascular surgery, 58(3): 659-665)

$$S = \frac{< A_0 > - A_s}{< A_0 >} = \frac{\Delta A}{< A_0 >}, \tag{43}$$

where: $\Delta A$ means changes in cross-sectional area of a lumen due to stenosis and S means degree of stenosis, which can be transposed into

$$\left(\frac{< A_0 >}{A_s} - 1\right)^2 = \left(\frac{S}{1 - S}\right)^2. \tag{44}$$

As a consequence, a nondimensional characteristic of the trans-stenotic resistance is given as

$$Eu_2 = k_s \left( \frac{S}{1-S} \right)^2, \qquad (45)$$

where $k_s$ is an empirical parameter which can be calculated using steady-state CFD simulations.

According to the invention, a pressure loss at transient or steady-state conditions (relating to $\Delta p_s$ or $\Delta P_s$, where $\Delta p_s$ is an instantaneous value of pressure loss and $\Delta P_s$ is a medium value of pressure loss) can be calculated using similarity flow invariants. This takes into consideration that in practice both above-mentioned pressure loss components are added together thereby arriving at $Eu = Eu_1 + Eu_2$ (see equations (40) and (45) above), so

$$Eu = \frac{B}{Re} \frac{l}{\langle d \rangle} + k_s \left( \frac{S}{1-S} \right)^2, \qquad (46)$$

and consequently, by introducing the Lagrange number we arrive at

$$La = Eu \cdot Re = \frac{\Delta p}{\langle v \rangle} \frac{\langle d \rangle}{\eta} = \frac{\pi}{4} \frac{\Delta p}{q} \frac{\langle d \rangle^3}{\eta}. \qquad (47)$$

This way we get a preferred embodiment of the flow characteristics describing $\Delta p_1 + \Delta p_2$

$$La = B \frac{l}{\langle d \rangle} + k_s \left( \frac{S}{1-S} \right)^2 Re. \qquad (48)$$

Equivalently, the following equation is obtained

$$La = B \frac{l}{\langle d \rangle} + K_s Re, \qquad (49)$$

where $K_s = k_s \cdot (S/(1-S))^2$ is an empirical parameter describing trans-stenotic level of dissipation. Benefit of this embodiment is pace of calculations originating from using linear equations instead of, for example, quadratic equations. This does not come with the cost of precision of results thereby obtained. In an embodiment, the cross-section is cylindrical and $B$=32.

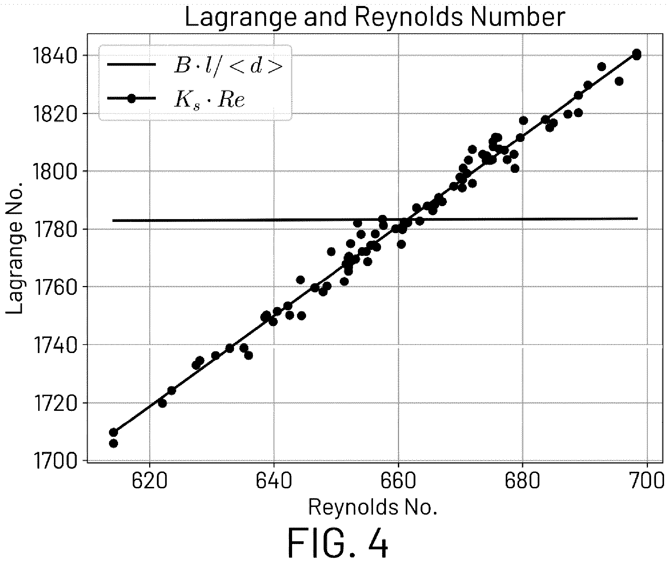

Based on the Lagrange and Reynolds numbers, "equivalent" diameter $\langle d \rangle$ and $K_s$ are computed as exemplified on Fig. 4. Every computation is performed using the medical data of the patient used for Fig. 3.

In another embodiment of the invention, the system of similarity invariants provides an universal relation describing the steady-state component $\Delta p_s$ with precision of the constant $\Delta p_0$. Insofar as there will be a need to reconstruct the full transient of the variation of a distal pressure $p_d$ as a function of time, it will have to be supplemented with a $\Delta p_t$ component. For avoidance of doubt, the "full transient" relates to a flow in which there is no reasonably observable trends in its change in time. Values of similarity invariants ($La, Re, Eu$) are calculated according to their generally known definitions, and pressure loss $\Delta p_s$ and flow $q$, empirical parameters like $B$ or $K_s$ can be obtained using steady-state CFD simulations (so, for example, for $B$ and $K_s$ only two calculations are needed which ensures faster arriving at the results). Diameter $\langle d \rangle$ can be selected to have the value which is equivalent to the one obtained from segmentation of the geometry model (and therefore surface area as $A = \pi \langle d \rangle^2 / 4$), while the dynamic coefficient of viscosity $\eta$ can be selected according to the patient-specific rheological properties of blood (Baskurt OK (2007) Handbook of hemorheology and hemodynamics, IOS Press). The skilled person knows that viscosity can be calculated using various known models used for non-Newtonian fluids, and for Newtonian fluid one can use a constant value of viscosity. For comparison, a typical state-of-the-art approach would require a few thousands of time-consuming CFD simulations.

In an embodiment of the invention, a diameter $\langle d \rangle$ does not directly reflect the diameter in terms of the segmentation result, but it is selected to be "equivalent" to the diameter obtained from segmentation results in terms of the level of energy dispersion. In this sense, the improved concept of the invention discards the idealized approach to the evaluation of trans-stenotic energy loss proposed by existing models (Schönfeld JC (1949), Young & Tsai (1973), Young & Tsai (1975), Young & Tsai (1977) and the most recent). Only in the case of large diameter arteries (as in Young & Tsai (1977)) or artificial models of geometry (and even more so of stenosis) in vitro or in silico will be possible to measure diameter of the vessel directly from the geometry. This limitation originates from too low resolution of experimental geometries which allows to be sufficiently precise only for large diameter arteries or for artificially simplified arteries (for example, arteries represented as simple tubes).

In a preferred embodiment of the invention, "equivalent" diameter $\langle d \rangle$ is considered to be the diameter of the circular channel, for which the energy loss under steady-state laminar flow conditions will be equal to the energy loss that will be produced under the actual geometry of the artery, under identical flow conditions. Under such circumstances, the hydrodynamic coefficient of friction $f$ and the Reynolds ($Re$) and Poiseuille ($Po$) similarity numbers satisfy the equation

$$f = \frac{64}{Re} \rightarrow f \cdot Re = 64 = const. \tag{50}$$

At the same time, as previously mentioned regarding pressure loss on straight or low curvature sections, $\Delta p = f \cdot l / \langle d \rangle \cdot \varrho \langle v \rangle^2 / 2$. Thus, the coefficient of friction is equal to

$$f = \frac{2 \langle d \rangle}{\varrho \langle v \rangle^2} \frac{\Delta p}{l}. \tag{51}$$

Noting that $Re = \langle v \rangle \langle d \rangle \varrho / \eta$ we receive the following form for the Poiseuille number

$$f \cdot Re = \frac{2 \langle d \rangle^2}{l \eta} \frac{\Delta p}{\langle v \rangle}, \tag{52}$$

or by replacing the "equivalent" velocity (v) with the flow rate

$$f \cdot Re = \frac{\pi \langle d \rangle^4}{2 \, l \eta} \frac{\Delta p}{q}, \tag{53}$$

with the ratio $\Delta p / q = R_0$ representing the resistance which, in this embodiment, is a constant value.

Therefore, in a preferred embodiment of the invention, an "equivalent" diameter $\langle d \rangle$ is considered to be the value satisfying the system of equations given below

$$\begin{cases} f \cdot Re = 64 \\ f \cdot Re = \dfrac{\pi \langle d \rangle^4}{2 \, l \, \eta} \dfrac{\Delta p}{q}. \end{cases} \tag{54}$$

This embodiment offers further improvement in pace of calculations while not jeopardizing its accuracy.

An embodiment of the invention comprises five steps: (1) selection of "equivalent" diameter $\langle d \rangle$, (2) approximation of viscous segment of flow characteristics, (3) approximation of local pressure loss segment of flow characteristics, (4) calculation of steady-state pressure drop, (5) calculation of transient (inertial) component of pressure drop. This is exemplified at Fig. 3, where $\langle d \rangle$ is equal to about 2.064 nm. For embodiment of Fig. 3, we can calculate <v> using $\langle d \rangle$ which is clear for the skilled person. The entire procedure is repeated for all locations and branches for which steady-state CFD simulation results are available.

STEP 1, preliminary to the reconstructing flow characteristics, involves selecting scales for describing an invariant flow by using similarity invariants. Of key importance here is a characteristic linear dimension scale and thus the equivalent diameter $\langle d \rangle$ as the velocity scale $\langle v \rangle$ is dependent on it. In more preferred embodiment, the scale is $\langle d \rangle$. In another embodiment, the scale is $\langle v \rangle$. According to the invention, the value of the equivalent diameter $\langle d \rangle$ is calculated as explained above to obtain consistency of dispersion phenomena between two cross-sections in an artery. In one example, the two cross-sections are proximal and distal cross-sections.

STEP 2 involves selecting description of the flow characteristics from those described herein in a segment of a coronary vessel relating to the viscous energy dissipation on straight sections and those with small curvature. In one embodiment, this includes using the flow characteristics defined in equation (32), (34) or (54).

Under real conditions (which for a human being are physiological conditions) a pressure loss is typically the sum of linear and local pressure loss. This has great significance in the case of flow in the coronary arteries, where the variability of geometry is high. In fact, it may be very difficult to separate the linear (strictly viscous) component from the local component. Instead of pursuing separation of pressure loss into the sum of linear and local pressure loss components, in a preferred embodiment of the invention, the values of the flow rate $q$ and, consequently, the velocity scale $\langle v \rangle$ and the

Reynolds number are chosen in order to have the second component of the flow characteristic negligibly small and therefore $B \cdot (l/\langle d \rangle) \gg K_s \cdot Re$. Formally, under idealized conditions of an embodiment, the value of the parameter $B$ is equal to 32. In other embodiments, the value of $B$ will vary. In one embodiment, $B$ is treated here as a constant at a fixed distal cross-section (distant from the inlet by 1). In this case, if in real conditions $K_s \neq 32$, then $K_s = const$ and only this part of the flow characteristic is considered at the step of determining this parameter.

STEP 3 refers to the second component of flow characteristics describing local losses. Its goal is to determine the value of the empirical parameter $K_s$. While there may be many stenotic components on a coronary artery, which is known from physiology, the predominant ones are stenoses formed by atherosclerotic plaques. If there are no functionally significant stenoses on a given branch, then in terms of physiological flow conditions, this component will not have any significant role. In an embodiment, the value of the $K_s$ parameter can be determined as the slope coefficient of a simple regression in the $La = f(Re)$ system, whose intercept is equal to $B \cdot l/\langle d \rangle$.

STEP 4, is to calculate the value of pressure loss $\Delta p_s$ using steady-state CFD simulations. Since $La = Eu \cdot Re$, the pressure loss $\Delta p_s$ can be calculated as $\Delta p_s = \varrho \langle v \rangle^2 \cdot La/Re$. This embodiment is applicable to calculation of *in silico* FFR This application originates from the fact that through STEP 4, an averaged values are obtained and such values are used in calculation of *in silico* FFR.

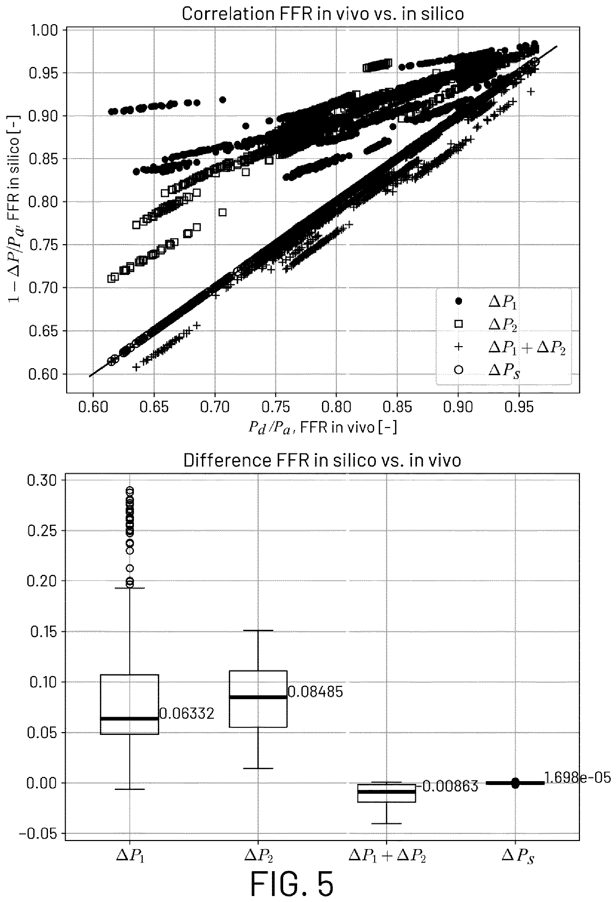

Fig. 5 summarizes the results of clinical trials. The clinical trials provided medical data of patients. The patients were 30 females (8 with myocardial ischemia) with mean heart rate $68.30 \pm 5.06$/min, mean systolic and diastolic blood pressure $131.83 \pm 11.03$ mmHg and $79.47 \pm 9.93$ mmHg respectively, and 25 males (17 with myocardial ischemia), mean heart rate $68.72 \pm 6.02$/min, mean systolic and diastolic blood pressure $136.72 \pm 13.46$ mmHg and $82.56 \pm 8.09$ mmHg respectively. The *in vivo* FFR ranged from 0.61 to 0.92. This medical data was used to compare experimental results with the ones obtained using the invention.

The medical data included 1960 human cardiac cycles for which *in vivo* pressure measurements were made with a pressure wire. For each of these cardiac cycles, the pressure loss (steady-state) was calculated by following STEP 1 through STEP 4 and then (STEP 5) the distal pressure was approximated. This allowed to calculate FFR (*in silico*) using only $\Delta p_s$ (steady-state pressure loss component in STEPs 1-4) and with a combination of $\Delta p_s$ and $\Delta p_t$ (transient pressure loss component, STEPs 1-5)

A correlation between *in vivo* FFR and *in silico* FFR was established. The correlation coefficient was 80.3% using only linear component of $\Delta p_s$ ($\Delta p_1$). The correlation coefficient was 93.8% using local resistance component ($\Delta p_2$), although the deviation was large and diagnostically unacceptable (+0.063 and +0.0848, respectively). However, when the pressure loss and FFR index were calculated solely based on the two components obtained after STEP 4 ($\Delta p_1 + \Delta p_2$), the correlation increased to 98.7% and the median difference -0.009. FFR values obtained for $\Delta p_1 + \Delta p_2$ were thus diagnostically acceptable, and had comparable or even smaller errors than the ones expected for *in vivo* FFR.

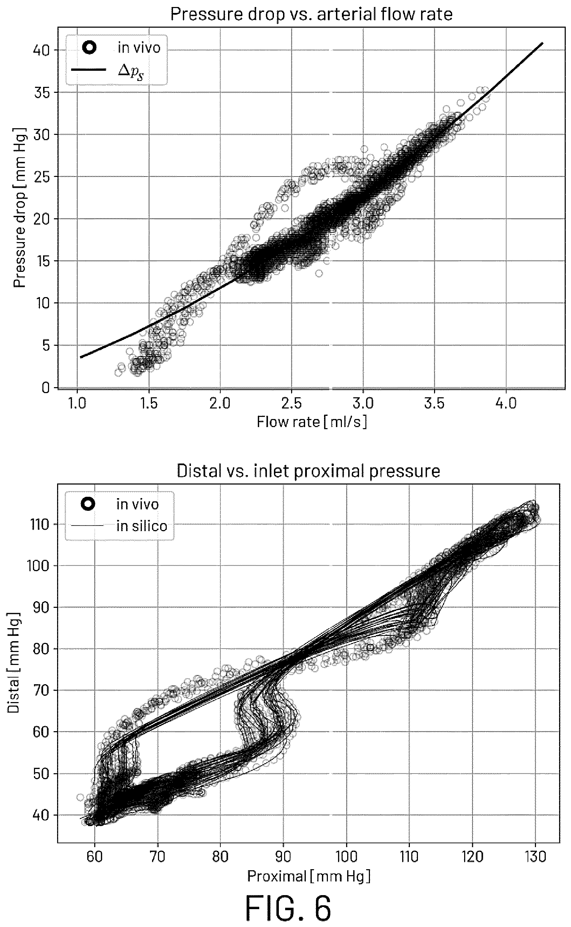

Fig. 6 presents an example of curves describing *in vivo* and *in silico* pressures obtained using the same medical data as used for Fig. 5. The curves obtained *in silico* reproduces the one obtained *in vivo*. Values of pressure drop vs. flow rate obtained *in silico* are reproducing values obtained *in vivo*.

STEP 5 is for reconstruction of pressure loss as a function of flow which includes variation thereof over time ($\Delta p = f(q(t))$). In this step, the steady-state pressure values $\Delta p_s$ obtained in STEP 4 are used and the transient pressure loss component $\Delta p_t$ is used. As described earlier, $\Delta p_t$ is calculated according to the second principle of dynamics $\varrho \, l \cdot d\langle v \rangle/dt$. In a preferred embodiment, the empirical coefficient is equal to $k_t = 1.2$.

The results of *in silico* FFR calculations obtained in STEP 5 were evaluated using the results obtained from clinical trials. The reference is made to Fig. 5. The degree of correlation is 99.9% and the median error is only $1.69813 \cdot 10^{-5}$.

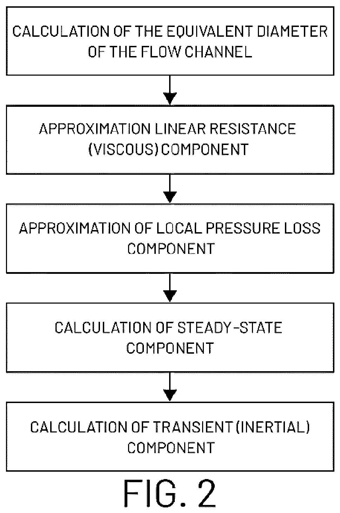

An embodiment implementing STEPS 1-5 is given on Fig. 2. In the first step, "equivalent" diameter (d) of the artery branch or flow channel is calculated. During steps two and three, approximation of linear resistance $\Delta p_1$ and local resistance $\Delta p_2$ is performed. In the fourth step steady-state component of pressure drop ($\Delta p_s$) is calculated, what allows to go to the last step, which is calculating transient component of pressure drop ($\Delta pt$). These five steps allow to assess pressure drop in a coronary artery including its transient component.

At any of the STEPs, the density ($\varrho$) and the dynamic viscosity coefficient ($\eta$) calculations can be done as described below. Basically, $\varrho$ and $\eta$ are patient-specific parameters. The density of blood, for example, can be approximated by Hawksley formula $\varrho = \varrho_{pl}(1 - HCT) + HCT \cdot \varrho_{RBC}$, where $\varrho_{pl}, \varrho_{RBC}$ are density of plasma and erythrocytes, respectively, and $HCT$ is a hematocrit number (De Gruttola S et al. (2005) Computational simulation of a non-newtonian model of the blood separation process, Artif Organs, 29(12): 949-59). There is also a wide variety of blood rheology models applicable here, for example, Carreau, Cross, Yasuda models, as well as many others (Yilmaz F, Gundogdu MY (2008) A critical review on blood flow in large arteries; relevance to blood rheology, viscosity models, and physiologic conditions, Korea-Australia Rheology Journal, 20(4): 197-211). While it would be possible to introduce a factor of individual variability in this way, in a preferred embodiment these parameters are chosen as constants. In this preferred embodiment, the accuracy of the calculations will not be significantly affected.

**Claims**

1. A computer-implemented method for calculation of a pressure drop *(Δp)* between a first cross-section and a second cross-section of an artery, wherein the second cross-section is downstream from the first cross-section and the two sections are separated by an artery channel having a length (*l*), and wherein the pressure drop *(Δp)* is expressed as $\Delta p = \Delta p_s$, with $\Delta p_s$ being a steady-state pressure loss component, wherein the method comprises calculating of the steady-state pressure loss component ($\Delta p_s$) using computational fluid dynamics (CFD) simulations, and wherein the computational fluid dynamics (CFD) simulations are steady-state computational fluid dynamics (CFD) simulations, and wherein $\Delta p_s$ is expressed as $\Delta p_s = \Delta p_0 + \Delta p_1 + \Delta p_2$, where $\Delta p_0$ is zero flow pressure, $\Delta p_1$ is viscous resistance for straight parts of the channel or parts of the channel with radius of curvature (R) to radius of the part (*r*) equal *r/R* < 10, $\Delta p_2$ is local resistance for parts of the channel with a stenosis, and wherein the viscous resistance ($\Delta p_1$) is equal to

$$\Delta p_1 = f \frac{l}{\langle d \rangle} \varrho \frac{\langle v \rangle^2}{2},$$

where *f* is hemodynamic friction coefficient, $\varrho$ is density of blood, and wherein $\langle d \rangle$ is "equivalent" inner diameter of the channel calculated for a model of the channel which has a round cross-section and length identical to the length of the channel for which the model is created and while assuming that pressure loss for a laminar flow is the same for the channel and for the model of the channel, and (v) is "equivalent" flow velocity calculated for the model of the channel while assuming that pressure loss for a laminar flow is the same for the channel and for the model of the channel, and wherein the local resistance ($\Delta p_2$) is proportional to $\varrho \frac{\langle v \rangle^2}{2}$ .

2. The computer-implemented method of claim 1, wherein the viscous resistance ($\Delta p_1$) is calculated using

$$Eu_1 = \frac{f}{2} \frac{l}{\langle d \rangle},$$

where Euler number is equal to $Eu_1 = \frac{\Delta p_1}{\varrho \cdot \langle v \rangle^2}$ .

3. The computer-implemented method of claim 2, wherein the Euler number is expressed as

$$Eu_1 = \frac{32}{Re} \frac{l}{\langle d \rangle}$$

and *Re* is expressed as

$$Re = \frac{\langle v \rangle \langle d \rangle \varrho}{\eta} = \frac{4}{\pi} \frac{q \varrho}{\langle d \rangle \eta},$$

where $\eta$ is dynamic viscosity coefficient, *q* is flow.

4. The computer-implemented method of any of claims 1-3, wherein the local resistance ($\Delta p_2$) is calculated using

$$Eu_2 = k_s \left( \frac{S}{1-S} \right)^2,$$

where $k_s$ is empirical parameter, and

$$S = \frac{<A_0> - A_s}{<A_0>} = \frac{\Delta A}{<A_0>}$$

where $\Delta A$ means changes in cross-sectional area of the channel due to stenosis and $S$ means degree of stenosis, $<A_0>$ is "equivalent" cross-sectional area calculated using "equivalent" diameter $\langle d \rangle$ that is proximal to the stenosis, and $A_s$ is cross-sectional area at the neck of the stenosis.

5. The computer-implemented method of any of claims 1-4, wherein the sum of the viscous resistance and the local resistance ($\Delta p_1 + \Delta p_2$) is calculated using the Lagrange number

$$La = 32 \frac{l}{\langle d \rangle} + k_s \left( \frac{S}{1-S} \right)^2 Re,$$

where $k_s$ is an empirical parameter, and

$$S = \frac{<A_0> - A_s}{<A_0>} = \frac{\Delta A}{<A_0>},$$

where $\Delta A$ means changes in cross-sectional area of the channel due to stenosis and S means degree of stenosis, $<A_0>$ is "equivalent" cross-sectional area calculated using "equivalent" diameter $\langle d \rangle$ that is proximal to the stenosis, and $A_s$ is cross-sectional area at the neck of the stenosis.

6. The computer-implemented method of any of claims 1-5, wherein "equivalent" diameter ($\langle d \rangle$) satisfies the following system of equations

$$\begin{cases} f \cdot Re = 64 \\ f \cdot Re = \frac{\pi \langle d \rangle^4}{2\,l\,\eta} \frac{\Delta p}{q}, \end{cases}$$

where $\eta$ is dynamic viscosity coefficient.

7. The computer-implemented method of any of claims 1-6, wherein the pressure drop ($\Delta p$) is defined as $\Delta p = \Delta p_s + \Delta p_t$, where $\Delta p_t$ being a transient pressure loss component, and $\Delta p_t$ is expressed as

$$\Delta p_t = k_t \frac{\varrho l}{<A_0>} \frac{dq}{dt}$$

where $t$ is time, $k_t$ is empirical index defined within the range 1 and 1.2, and $<A_0>$ is "equivalent" surface area calculated using $\langle d \rangle$, and the method includes calculating of the transient pressure loss component ($\Delta p_t$).

8. The computer-implemented method of any of claims 1-7, wherein the artery is a coronary artery, carotid artery, renal artery, peripheral artery, or aorta.

9. The computer-implemented method of any of claims 1-8, wherein $q$ is within the range of 1 ml/s to 15 ml/s, or $q$ is equal to 1.5 ml/s, 3.5 ml/s, or 5 ml/s, or where $q$ is within the range $q_0/5$ to $3q_0$ where $q_0$ is a typical value of flow for the artery.

10. The computer-implemented method of any of claims 1-9, wherein the two cross-sections or the artery channel between the two cross-sections have identical or similar topology, wherein similar topology is defined using a similarity index which takes into account change in internal diameter between the two cross-sections or across the channel between the two cross-sections, branching at the two cross-sections or across the channel between the two cross-sections or all of the above, and wherein the similarity index has a value of at least 50%, 55%, 60%, 65%, 70%, 75%, 80%, 85%, 90%, 95% or 99%.

11. The computer-implemented method of any of claims 1-10, wherein $r/R$ is below 9, 8, 7, or 6.

12. The computer-implemented method of any of claims 1-11, wherein the method further includes calculating of a fractional flow reserve (FFR).

**13.** A computer program [product] comprising instructions which, when the program is executed by a computer, cause the computer to carry out the method of any of claims 1-12.

**14.** A computer-readable [storage] medium comprising instructions which, when executed by a computer, cause the computer to carry out the method of any of claims 1-12.

**Patentansprüche**

**1.** Ein computerimplementiertes Verfahren zur Berechnung eines Druckabfalls ($\Delta p$) zwischen einem ersten Querschnitt und einem zweiten Querschnitt einer Arterie, wobei der zweite Querschnitt stromabwärts des ersten Querschnitts angeordnet ist und die beiden Querschnitte durch einen Arterienkanal mit einer Länge ($l$) voneinander getrennt sind, und wobei der Druckabfall ($\Delta p$) als $\Delta p = \Delta p_s$, mit $\Delta p_s$ asugedrückt wird, wobei die eine stationäre Druckverlustkomponente ist, wobei das Verfahren die Berechnung der stationären Druckverlustkomponente ($\Delta p_s$) unter Verwendung von CFD-Simulationen (Computational Fluid Dynamics) umfasst, und wobei die CFD-Simulationen (Computational Fluid Dynamics) stationäre CFD-Simulationen sind, und wobei $\Delta p_s$ als $\Delta p_s = \Delta p_0 + \Delta p_1 + \Delta p_2$ ausgedrückt wird, wobei der Fließdruck Null $\Delta p_0$ ist, der viskose Widerstand für $\Delta p_1$ gerade Teile des Kanals oder Teile des Kanals mit einem Krümmungsradius (R) bis zum Radius des Teils (*r*) *gleich r/R* < 10 $\Delta p_2$ ist, der lokale Widerstand für Teile des Kanals mit einer Stenose ist, und wobei der viskose Widerstand ($\Delta p_1$) gleich ist

$$\Delta p_1 = f \frac{l}{\langle d \rangle} \varrho \frac{\langle v \rangle^2}{2},$$

wobei $f$ der hämodynamische Reibungskoeffizient ist, $\varrho$ die Dichte des Blutes ist und wobei $\langle d \rangle$ der "äquivalente" Innendurchmesser des Kanals ist, der für ein Modell des Kanals berechnet wird, das einen runden Querschnitt und eine Länge hat, die mit der Länge des Kanals identisch ist, für den das Modell erstellt wird, und unter der Annahme, dass der Druckverlust für eine laminare Strömung für den Kanal und für das Modell des Kanals gleich ist, und $\langle v \rangle$ eine "äquivalente" Strömungsgeschwindigkeit ist, die für das Modell des Kanals berechnet wird, unter der Annahme, dass der Druckverlust für eine laminare Strömung für den Kanal und für das Modell des Kanals gleich ist, und wobei der lokale Widerstand proportional ($\Delta p_2$) zu

$$\varrho \frac{\langle v \rangle^2}{2}$$

ist.

**2.** Das computerimplementierte Verfahren nach Anspruch 1, wobei der viskose Widerstand ($\Delta p_1$) unter Verwendung von

$$Eu_1 = \frac{f}{2} \frac{l}{\langle d \rangle},$$

berechnet wird, wobei die Euler-Zahl $Eu_1 = \frac{\Delta p_1}{\varrho \cdot \langle v \rangle^2}$ gleich ist.

**3.** Das computerimplementierte Verfahren nach Anspruch 2, wobei die Euler-Zahl ausgedrückt wird als

$$Eu_1 = \frac{32}{Re} \frac{l}{\langle d \rangle}$$

und *Re* ausgedrückt wird als

$$Re = \frac{\langle v \rangle \langle d \rangle \varrho}{\eta} = \frac{4}{\pi} \frac{q\varrho}{\langle d \rangle \eta},$$

wobei $\eta$ der dynamische Viskositätskoeffizient die Strömung $q$ ist.

4. Das computerimplementierte Verfahren nach einem der Ansprüche 1-3, wobei der lokale Widerstand ($\Delta p_2$) unter Verwendung von

$$Eu_2 = k_s \left(\frac{S}{1-S}\right)^2,$$

wobei $k_s$ ein empirischer Parameter ist, und

$$S = \frac{<A_0> - A_s}{<A_0>} = \frac{\Delta A}{<A_0>}$$

wobei $\Delta A$ die Änderungen der Querschnittsfläche des Kanals aufgrund einer Stenose bedeutet und $S$ den Grad der Stenose bedeutet, $<A_0>$ die "äquivalente" Querschnittsfläche ist, die unter Verwendung des "äquivalenten" Durchmessers $\langle d \rangle$ berechnet wird, der proximal zur Stenose liegt, und $A_s$ die Querschnittsfläche am Hals der Stenose ist.

5. Das computerimplementierte Verfahren nach einem der Ansprüche 1-4, wobei die Summe des viskosen Widerstands und des lokalen Widerstands ($\Delta p_1 + \Delta p_2$) unter Verwendung der Lagrange-Zahl berechnet wird

$$La = 32\frac{l}{\langle d \rangle} + k_s \left(\frac{S}{1-S}\right)^2 Re,$$

wobei $k_s$ ein empirischer Parameter ist, und

$$S = \frac{<A_0> - A_s}{<A_0>} = \frac{\Delta A}{<A_0>},$$

wobei $\Delta A$ die Änderungen der Querschnittsfläche des Kanals aufgrund einer Stenose bedeutet und $S$ den Grad der Stenose bedeutet, $<A_0>$ die "äquivalente" Querschnittsfläche ist, die unter Verwendung des "äquivalenten" Durchmessers $\langle d \rangle$ berechnet wird, der proximal zur Stenose liegt, und $A_s$ die Querschnittsfläche am Hals der Stenose ist.

6. Das computerimplementierte Verfahren nach einem der Ansprüche 1-5, wobei der "äquivalente" Durchmesser ($\langle d \rangle$) das folgende Gleichungssystem erfüllt

$$\begin{cases} f \cdot Re = 64 \\ f \cdot Re = \frac{\pi \langle d \rangle^4}{2\, l\, \eta}\frac{\Delta p}{q}, \end{cases}$$

wobei $\eta$ der dynamische Viskositätskoeffizient ist.

7. Das computerimplementierte Verfahren nach einem der Ansprüche 1-6, wobei der Druckabfall *(Δp)* als $\Delta p = \Delta p_s + \Delta p_t$ *definiert ist,* wobei $\Delta p_t$ eine transiente Druckverlustkomponente ist, und $\Delta p_t$ ausgedrückt wird als

$$\Delta p_t = k_t \frac{\varrho l}{<A_0>}\frac{dq}{dt}$$

wobei $t$ die Zeit ist, $k_t$ ein empirischer Index ist, der innerhalb des Bereichs 1 und 1.2 definiert ist, und $<A_0>$ eine "äquivalente" Oberfläche ist $\langle d \rangle$, die unter Verwendung von $\langle d \rangle$, berechnet wird, und das Verfahren die Berechnung der transienten Druckverlustkomponente ($\Delta p_t$) beinhaltet.

8. Das computerimplementierte Verfahren nach einem der Ansprüche 1-7, wobei die Arterie eine Koronararterie, Karotisarterie, Nierenarterie, periphere Arterie oder Aorta ist.

9. Das computerimplementierte Verfahren nach einem der Ansprüche 1-8, wobei $q$ innerhalb des Bereichs von 1 ml/s bis 15 ml/s liegt oder q gleich 1,5 ml/s, 3,5 ml/s oder 5 ml/s ist oder wobei innerhalb des Bereichs von $q_0/5$ bis $3q_0$ liegt, wobei $q_0$ ein typischer Wert der Strömung für die Arterie ist.

10. Das computerimplementierte Verfahren nach einem der Ansprüche 1-9, wobei die zwei Querschnitte oder der Arterienkanal zwischen den zwei Querschnitten eine identische oder ähnliche Topologie aufweisen, wobei eine ähnliche Topologie unter Verwendung eines Ähnlichkeitsindex definiert ist, der eine Änderung des Innendurchmessers zwischen den zwei Querschnitten oder über den Kanal zwischen den zwei Querschnitten berücksichtigt, sich an den zwei Querschnitten oder über den Kanal zwischen den zwei Querschnitten oder allen der oben genannten verzweigt, und wobei der Ähnlichkeitsindex einen Wert von mindestens 50 %, 55 %, 60 %, 65 %, 70 %, 75 %, 80 %, 85 %, 90 %, 95 % oder 99 % aufweist.

11. Das computerimplementierte Verfahren nach einem der Ansprüche 1-10, wobei $r/R$ unter 9, 8, 7 oder 6 liegt.

12. Das computerimplementierte Verfahren nach einem der Ansprüche 1-11, wobei das Verfahren ferner Berechnen einer fraktionalen Strömungsreserve (Fractional Flow Reserve - FFR) beinhaltet.

13. Ein Computerprogramm [Produkt], das Anweisungen beinhaltet, die, wenn das Programm von einem Computer ausgeführt wird, den Computer veranlassen, das Verfahren nach einem der Ansprüche 1-12 durchzuführen.

14. Ein computerlesbares [Speicher-]Medium, das Anweisungen beinhaltet, die, wenn sie von einem Computer ausgeführt werden, den Computer veranlassen, das Verfahren nach einem der Ansprüche 1-12 durchzuführen.

**Revendications**

1. Procédé mis en œuvre par l'ordinateur pour calculer une chute de pression ($\Delta$p) entre une coupe transversale première et une seconde coupe transversale d'une artère, dans lequel la seconde coupe transversale est en aval de la première coupe transversale et deux coupes sont séparées par un canal artériel d'une longueur ($l$), et dans lequel la chute de pression ($\Delta$p) est exprimée en tant que $\Delta p = \Delta p_s$, $\Delta p_s$ étant une composante de perte de pression en régime permanent, le procédé comprenant le calcul de la composante de perte de pression en régime permanent $\Delta p_s$, en utilisant des simulations de mécanique des fluides numérique (CFD), et dans lequel des simulations de mécanique des fluides numérique (CFD) sont des simulations de mécanique des fluides numérique (CFD) en régime permanent, et dans lequel $\Delta p_s$ est exprimée en tant que $\Delta p_s = \Delta p_0 + \Delta p_1 + \Delta p_2$, où $\Delta p_0$ est la pression à débit nul, $\Delta p_1$ est la force de viscosité pour des parties droites ou des parties du canal d'un rayon de courbure ($R$) à un rayon de partie ($r$) égal à $r/R <$ 10, $\Delta p_2$ est une résistance local pour des parties du canal avec une sténose, et dans lequel la force de viscosité est égal à :

$$\Delta p_1 = f \frac{l}{\langle d \rangle} \varrho \frac{\langle v \rangle^2}{2}$$

où $f$ est le coefficient de frottement hémodynamique, $\varrho$ est une densité du sang, et dans laquelle $\langle d \rangle$ est un diamètre interne « équivalent » du canal calculé pour un modèle du canal qui présente une coupe transversale ronde et une longueur identique à une longueur du canal pour lequel le modèle est crée et tout en assumant que la chute de pression pour l'écoulement laminaire est la même pour le canal et pour le modèle du canal, et $\langle v \rangle$ est une vitesse d'écoulement « équivalente » calculée pour le modèle du canal tout en assumant que la chute de pression pour l'écoulement laminaire est la même pour le canal et pour le modèle du canal, et dans laquelle la résistance locale ($\Delta p_2$) est proportionnelle à $\varrho \frac{\langle v \rangle^2}{2}$ .

2. Procédé mis en œuvre par ordinateur selon la revendication 1, dans lequel la force de viscosité ($\Delta p_1$) est calculée en utilisant

$$Eu_1 = \frac{f}{2} \frac{l}{\langle d \rangle},$$

où le nombre d'Euler est égal à $Eu_1 = \frac{\Delta p_1}{\varrho \cdot \langle v \rangle^2}$

3. Procédé mis en œuvre par ordinateur selon la revendication 2, dans lequel le nombre d'Euler est exprimé en tant que :

$$Eu_1 = \frac{32}{Re} \frac{l}{\langle d \rangle}$$

et *Re* est exprimé en tant que

$$Re = \frac{\langle v \rangle \langle d \rangle \varrho}{\eta} = \frac{4}{\pi} \frac{q\varrho}{\langle d \rangle \eta},$$

où $\eta$ est le coefficient de viscosité dynamique, *q* est l'écoulement.

4. Procédé mis en œuvre par ordinateur selon l'une quelconque des revendications 1-3, dans lequel la résistance locale ($\Delta p_2$) est calculée en utilisant

$$Eu_2 = k_s \left( \frac{S}{1-S} \right)^2,$$

où $k_s$ est un paramètre empirique, et

$$S = \frac{\langle A_0 \rangle - A_s}{\langle A_0 \rangle} = \frac{\Delta A}{\langle A_0 \rangle}$$

où $\Delta A$ signifie des évolutions dans l'aire de coupe transversale du canal causées par la sténose et *S* signifie le degré de sténose, $\langle A_0 \rangle$ est l'aire de coupe transversale « équivalente » calculée en utilisant le diamètre « équivalent » $\langle d \rangle$ qui est proximal à la sténose, et $A_s$ est l'aire de coupe transversale au collet de sténose.

5. Procédé mis en œuvre par ordinateur selon l'une quelconque des revendications 1-4, dans lequel la somme de la force de viscosité et la résistance locale ($\Delta p_1 + \Delta p_2$) est calculée en utilisant le nombre de Lagrange

$$La = 32 \frac{l}{\langle d \rangle} + k_s \left( \frac{S}{1-S} \right)^2 Re,$$

où $k_s$ est un paramètre empirique, et

$$S = \frac{\langle A_0 \rangle - A_s}{\langle A_0 \rangle} = \frac{\Delta A}{\langle A_0 \rangle}$$

où $\Delta A$ signifie des évolutions dans l'aire de coupe transversale du canal causées par la sténose et *S* signifie le degré de sténose, $\langle A_0 \rangle$ est l'aire de coupe transversale « équivalente » calculée en utilisant le diamètre « équivalent » $\langle d \rangle$ qui est proximal à la sténose, et $A_s$ est l'aire de coupe transversale au collet de sténose.

6. Procédé mis en œuvre par ordinateur selon l'une quelconque des revendications 1-5, dans lequel le diamètre « équivalent » ($\langle d \rangle$) satisfait le système d'équations suivant :

$$\begin{cases} f \cdot Re = 64 \\ f \cdot Re = \dfrac{\pi \langle d \rangle^4}{2l\eta} \dfrac{\Delta p}{q}, \end{cases}$$

où $\eta$ est le coefficient de viscosité dynamique.

7. Procédé mis en œuvre par ordinateur selon l'une quelconque des revendications 1-6, dans lequel la chute de pression ($\Delta p$) est définie en tant que $\Delta p = \Delta p_s + \Delta p_t$, $\Delta p_t$ étant une composante de perte de pression transitoire, et $\Delta p_t$ est exprimé en tant que :

$$\Delta p_t = k_t \frac{\varrho l}{\langle A_0 \rangle} \frac{dq}{dt}$$

où $t$ est le temps, $k_t$ est l'indice empirique défini dans une plage comprise entre 1 et 1.2, et $\langle A_0 \rangle$ est une superficie « équivalente » calculée en utilisant $\langle d \rangle$, et le procédé comprend le calcul de la composante de perte de pression transitoire ($\Delta p_t$).

8. Procédé mis en œuvre par ordinateur selon l'une quelconque des revendications 1-6, dans lequel l'artère est une artère coronaire, une artère carotide, une artère rénale, une artère périphérique, ou une aorte.

9. Procédé mis en œuvre par ordinateur selon l'une quelconque des revendications 1-8, dans lequel $q$ est compris entre 1ml/s à 15 ml/s, ou $q$ est égal à 1.5ml/s, 3.5ml/s, ou 5ml/s, ou dans lequel $q$ est compris entre $q_0/5$ à $3q_0$, où $q_0$ est une valeur typique d'écoulement pour l'artère.

10. Procédé mis en œuvre par ordinateur selon l'une quelconque des revendications 1-9, dans lequel deux coupes transversales ou le canal artériel entre deux coupes transversales présente la topologie identique ou similaire, dans lequel la topologie similaire est définie en utilisant l'indice de similarité qui prend en compte l'évolution dans entre deux coupes transversales ou à travers le canal entre deux coupes transversales, branchant sur les deux coupes transversales ou à travers le canal entre deux coupes transversales ou tous les susmentionnés, et dans lequel l'indice de similarité présente une valeur d'au moins 50%, 55%, 60%, 65%, 70%, 75%, 80%, 85%, 90%, 95% ou 99%.

11. Procédé mis en œuvre par ordinateur selon l'une quelconque des revendications 1-10, dans lequel $r/R$ est inférieur à 9, 8, 7, ou 6.

12. Procédé mis en œuvre par ordinateur selon l'une quelconque des revendications 1-11, dans lequel le procédé en poutre comprend le calcul d'une réserve coronaire (FFR).

13. Produit programme d'ordinateur comprenant des instructions qui, lorsque le produit programme est exécuté par un ordinateur, amènent l'ordinateur à exécuter les étapes du procédé tel que défini dans l'une quelconque des revendications 1-12.

14. Support de stockage lisible par ordinateur comprenant des instructions qui, lorsqu'elles sont exécutées par un ordinateur, amènent l'ordinateur à exécuter les étapes du procédé tel que défini dans l'une quelconque des revendications 1-12.

(A)

(B)

$l$

$<d>$

(C)

Poiseuille No.

$B = const.$

$Po(<d>)$

Diameter $<d>$ [mm]

(D)

Lagrange No.

$B \cdot l / <d>$

$Ks \cdot Re$

Reynolds No.

(E)

Pressure drop [mmHg]

transient

steady-state

Flow rate [ml/s]

# FIG. 1

```
┌─────────────────────────────────────────┐
│  CALCULATION OF THE EQUIVALENT DIAMETER  │
│         OF THE FLOW CHANNEL              │
└─────────────────────────────────────────┘
                    │
                    ▼
┌─────────────────────────────────────────┐
│   APPROXIMATION LINEAR RESISTANCE        │
│        (VISCOUS) COMPONENT               │
└─────────────────────────────────────────┘
                    │
                    ▼
┌─────────────────────────────────────────┐
│   APPROXIMATION OF LOCAL PRESSURE LOSS   │
│             COMPONENT                    │
└─────────────────────────────────────────┘
                    │
                    ▼
┌─────────────────────────────────────────┐
│     CALCULATION OF STEADY-STATE          │
│             COMPONENT                    │
└─────────────────────────────────────────┘
                    │
                    ▼
┌─────────────────────────────────────────┐
│  CALCULATION OF TRANSIENT (INERTIAL)     │
│             COMPONENT                    │
└─────────────────────────────────────────┘
```

# FIG. 2

FIG. 3

Lagrange and Reynolds Number

FIG. 4

EP 4 629 887 B1

FIG. 5

FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 10373700 B2
- US 10354744 B2
- US 20190183579 A1

- WO 2020048642 A1
- EP 3820357 B1

### Non-patent literature cited in the description

- **GRACE SF**. Oscillatory motion of viscous liquid in a long straight tube. *The London, Edinburgh, and Dublin Philosophical Magazine and Journal of Science*, 1928, vol. 5 (31), 933-939
- **SCHÖNFELD JC**. Resistance and inertia of the flow of liquids in a tube or open channel. *Flow Turbulence and Combustion*, 1949, vol. 1 (1), 169-197
- **BOURASSA MG**. The history of cardiac catheterization. *The Canadian Journal of Cardiology*, 2005, vol. 21 (12), 1011-1014
- **YOUNG DF**; **TSAI FY**. Flow characteristics in models of arterial stenoses. II. *Unsteady flow, Journal of Biomechanics*, 1973, vol. 6 (5), 547-559
- **YOUNG DF**; **CHOLVIN NR**; **ROTH AC**. Pressure drop across artificially induced stenoses in the femoral arteries of dogs. *Circulation Research*, 1975, vol. 36 (6), 735-743
- **YOUNG DF et al.** Hemodynamics of arterial stenoses at elevated flow rates. *Circulation Research*, 1977, vol. 41 (1), 99-107
- **ITZCHAK Y et al.** Determination of the pressure drop across an arterial stenosis utilizing angiocinedensitometry. *The Yale Journal of Biology and Medicine*, 1977, vol. 50 (4), 375-381
- **GOULD KL**. Pressure-flow characteristics of coronary stenoses in unsedated dogs at rest and during coronary vasodilation. *Circulation Research*, 1978, vol. 43 (2), 242-253
- **MATES RE et al.** Fluid dynamics of coronary artery stenosis. *Circulation research*, 1978, vol. 42 (1), 152-162
- **MCMAHON MM et al.** Quantitative coronary angiography: measurement of the "critical" stenosis in patients with unstable angina and single-vessel disease without collaterals. *Circulation*, 1979, vol. 60 (1), 106-113
- **BESSEMS DD**. On the propagation of pressure and flow waves through the patient specific arterial system. *PhD thesis*, 2007

- **LIANG F et al.** Multi-scale modelling of the human cardiovascular system with applications to aortic valvular and arterial stenoses. *Medical & Biological Engineering & Computing*, 2009, vol. 47 (7), 743-755
- **HUO Y et al.** A validated predictive model of coronary fractional flow reserve. *Journal of the Royal Society, Interface*, 2012, vol. 9 (71), 1325-1338
- **ITU L et al.** Non-invasive hemodynamic assessment of aortic coarctation: validation with in vivo measurements. *Annals of Biomedical Engineering*, 2013, vol. 41 (4), 669-681
- 2021 ACC/AHA/SCAI Guideline for Coronary Artery Revascularization: Executive Summary: A Report of the American College of Cardiology. **LAWTON JS et al.** Circulation. American Heart Association, 2022, vol. 145, e4-e17
- **KNUUTI J et al.** 2019 ESC Guidelines for the diagnosis and management of chronic coronary syndromes. *European heart journal*, 2020, vol. 41 (3), 407-477
- **COOK CM et al.** Diagnostic accuracy of computed tomography-derived fractional flow reserve: A systematic review. *JAMA cardiology*, 2017, vol. 2 (7), 803-810
- **DRIESSEN RS et al.** Comparison of coronary computed tomography angiography, fractional flow reserve, and perfusion imaging for ischemia diagnosis. *Journal of the American College of Cardiology*, 2019, vol. 73 (2), 161-173
- **TORII R**; **YACOUB MH**. CT-based fractional flow reserve: development and expanded application. *Global cardiology science & practice*, 2021, E202120
- **PONTONE G et al.** Clinical applications of cardiac computed tomography: a consensus paper of the European Association of Cardiovascular Imaging-part II. *European heart journal, Cardiovascular Imaging*, 2022, vol. 23 (4), e136-e161

- **COURANT R** ; **FRIEDRICHS K** ; **LEWY H**. Über die partiellen Differenzengleichungen der mathematischen Physik. *Mathematische Annalen*, 1928, vol. 100, 32-74
- **DE MOURA CA** ; **KUBRUSLY CS**. The Courant-Friedrichs-Lewy (CFL) condition: 80 years after its discovery. *Birkhauser Boston*, 2013
- Normal hemodynamic state. **MILNOR WR**. Hemodynamics. Williams&Wilkins, 1982
- Pressure-flow relationships of the coronary arteries. **HOFFMAN JIE**. Coronary Circulation. Springer-Verlag, 1990, 109-125
- **PATEL N et al.** The zero-flow pressure measured immediately after a primary percutaneous coronary intervention for a ST-segment elevation myocardial infarction provides the most useful invasive index for predicting the extent of myocardial infarction after 6 months as showed in An OxAMI Study (Oxford Acute Myocardial Infarction). *JACC, Cardiovascular interventions*, 2015, vol. 8 (11), 1410-1421
- **FREIDOONIMEHR et al.** Effect of artery curvature on the coronary fractional flow reserve. *Physics of Fluids*, 2021, vol. 33, 031906
- **BIRD RB** ; **STEWART WE** ; **LIGHTFOOT EN**. Transport phenomena. John Wiley & Sons, 2007
- **WHITE FM**. Fluid Mechanics. WCB/McGraw-Hill, 1999
- **CARNICELLI AP et al.** Cross-sectional area for the calculation of carotid artery stenosis on computed tomographic angiography. *Journal of vascular surgery*, 2013, vol. 58 (3), 659-665
- **BASKURT OK**. Handbook of hemorheology and hemodynamics. IOS Press, 2007
- **DE GRUTTOLA S et al.** Computational simulation of a non-newtonian model of the blood separation process. *Artif Organs*, 2005, vol. 29 (12), 949-59
- **YILMAZ F** ; **GUNDOGDU MY**. A critical review on blood flow in large arteries; relevance to blood rheology, viscosity models, and physiologic conditions. *Korea-Australia Rheology Journal*, 2008, vol. 20 (4), 197-211